(19) Europäisches Patentamt
European Patent Office
Office européen des brevets

(11) EP 4 032 988 A1

(12) EUROPEAN PATENT APPLICATION

(43) Date of publication:
**27.07.2022 Bulletin 2022/30**

(21) Application number: **21305080.0**

(22) Date of filing: **22.01.2021**

(51) International Patent Classification (IPC):
***C12Q 1/6886*** *(2018.01)*

(52) Cooperative Patent Classification (CPC):
**C12Q 1/6886;** C12Q 2600/112; C12Q 2600/158

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institut Gustave Roussy**
**94800 Villejuif (FR)**

(72) Inventor: **The designation of the inventor has not yet been filed**

(74) Representative: **Santarelli**
**49, avenue des Champs-Elysées**
**75008 Paris (FR)**

# (54) METHOD FOR IDENTIFYING HARD-TO-TREAT OSTEOSARCOMA PATIENTS AT DIAGNOSIS AND IMPROVING THEIR OUTCOME BY PROVIDING NEW THERAPY

(57) The present invention provides tools for stratifying patients suffering from osteosarcoma, in order to identify patients having poor prognosis tumors. The invention also pertains to the use of an inhibitor of the PPARγ pathway as an antineoplastic treatment to improve the overall survival of patients with a poor prognosis osteosarcoma.

EP 4 032 988 A1

## Description

### FIELD OF THE INVENTION

The present invention relates to the field of anticancer treatment. In particular, the present invention concerns the prognosis of osteosarcoma in an individual, and provides an *in vitro* method for determining this prognosis, as well as diagnostic kits to perform this method. Also described herein is the use of an inhibitor of the PPARγ pathway as an antineoplastic treatment to improve the overall survival of patients with a poor prognosis osteosarcoma.

### BACKGROUND OF THE INVENTION

Osteosarcoma, the most common primary bone cancer in adolescents and young adults, presents a highly heterogeneous genomic and transcriptomic landscape as a result of multiple chromosomal rearrangements[1,2]. Such heterogeneity has complicated exploratory studies to determine the key oncogenic drivers underlying disease emergence or progression, and no routinely used prognostic biomarkers or robust stratification has been defined so far. As a result, there have been no major changes to treatments for 40 years, and a third of patients with osteosarcoma still experience treatment failure, primarily with metastatic relapses[3-7]. Over the last 20 years, the focus has been on genetic exploration of the disease by different DNA analysis techniques from Comparative Genomic Hybridization array (CGH)[8-12] to Whole Exome (WES)[1,2,13-16] or Genome Sequencing (WGS)[17-19]. These have reported numerous genetic events (mainly Copy Number Variations, CNV) related to slight/medium tumor fitness increase without any clear tumor stratification.

It has recently been demonstrated that despite common phenotypic features, primary osteosarcoma is highly polyclonal under near neutral selection[20]. These observations raise the possibility that the major phenotypic osteosarcoma traits are an emergent characteristic from the epigenetic or/and transcriptional reprogramming of heterogeneous genomic rearrangements in a polyclonal community. In other words, progression is a consequence of tumor plasticity.

In addition, in the highly constrained bone environment of osteosarcoma, it is challenging to evaluate tumor cell co-evolution with the tumor microenvironment (TME) composition and stromal cell proportions or activation states[21,22].

### SUMMARY OF THE INVENTION

To better understand the mechanisms underlying osteosarcoma and thus enrich the armamentarium against this disease, the inventors reasoned that, the transcriptomic landscape could overcome the genetic complexity of this disease and provide more interpretable information. With unsupervised machine learning strategy, they defined the repertoire of gene components describing osteosarcoma tumor clones and TME. They observed that component interactions through co-expression stratify the cohort into good and poor prognosis tumors.

Functional characterization of the components associates good prognosis tumors with specific innate immune expression and poor prognosis tumors with angiogenic, osteoclastic, and adipogenic activities, with distinct CNVs specific to each group. These distinct functional characteristics can be used to stratify treatment in osteosarcomas, for instance immune modulation (e.g. mifamurtide) for G1 group tumors (good prognosis), and anti-osteoclastic and anti-angiogenic therapies for G2 (hard-to-treat) group.

The inventors also identified underlying biological pathways of osteosarcoma, involving PPARγ, piRNA or CTAs which highlight new actionable targets.

Finally, they identified a panel of 37 genes involved in osteosarcoma and confirmed the predictive power of a minimal prognostic signature of 15 genes.

According to a first aspect, the present invention thus relates to an *in vitro* method for determining the prognosis of osteosarcoma in an individual, comprising measuring the expression levels of a collection of signature genes from a biological sample taken from said individual, applying the expression levels measured to a predictive model associating expression levels of said collection of signature genes with osteosarcoma outcome, and evaluating the output of said predictive model to determine prognosis of osteosarcoma in said individual.

The invention also pertains to a diagnostic kit for predicting the progression of osteosarcoma in a subject by measuring the level of expression of a collection of signature genes from a biological sample taken from the individual.

Another object of the present invention is the use of a PPARγ inhibitor for treating patients with a poor prognosis of osteosarcoma.

In particular, the invention provides an *in vitro* method for determining prognosis of osteosarcoma in an individual, comprising: (a) measuring expression levels of a collection of signature genes from a biological sample taken from said individual, wherein said collection of signature genes comprises at least two genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, GAGE12G, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4; (b) applying the expression levels measured in step (a) to a predictive model relating expression levels of said collection of signature genes with osteosarcoma outcome; and (c) evaluating the output of said predictive model to determine prognosis of osteosarcoma in said individual. In some embodiments, the collection of signature genes comprises CCDC34 and MEAF6. In some embodiments, the collection of signature genes comprises at least 5, preferably at least 7, more preferably at least 8 genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, GAGE12G, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4. In some embodiments, the collection of signature genes comprises AMZ2P1, C5orf28, CCDC34, GAGE12D, MEAF6, SLC2A6, SLC7A4 and THAP9-AS1. In some embodiments, the collection of signature genes comprises at least 10, preferably at least 12, more preferably at least 15 genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, GAGE12G, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4. In some embodiments, the collection of signature genes comprises AMZ2P1, C5orf28, CCDC34, ESCO1, GAGE12D, HADHA, MEAF6, RALGAPA2, SLC2A6, SLC7A4, THAP9-AS1 and TIMP3. In some embodiments, the collection of signature genes comprises AMZ2P1, ASXL2, C11orf58, C5orf28, CCDC34, ESCO1, GAGE12D, HADHA, MEAF6, PAIP1, RALGAPA2, SLC2A6, SLC7A4, THAP9-AS1 and TIMP3. In some embodiments, the collection of signature genes comprises AMZ2P1, C5orf28, CCDC34, ESCO1, FAM35DP, GAGE12D, HADHA, HIST2H2AA3, HIST2H2AA4, MAPK1, MEAF6, POLR2C, RALGAPA2, SLC2A6, SLC7A4, ST7L, THAP9-AS1, TIMP3 and TTPAL.

In some embodiments, the expression levels of said collection of signature genes are measured at diagnosis. In some embodiments, the expression levels of said collection of signature genes are measured at relapse.

In some embodiments, the gene expression levels of said signature genes are combined with one or more other parameters to predict progression of osteosarcoma in said individual. In some embodiments, the one or more other parameters are selected from the group consisting of drugs administered to the patient, age, tumour height and stage at diagnosis, presence or absence of metastasis at diagnosis and any combinations thereof.

In some embodiments, the biological sample is an osteosarcoma biopsy sample from the individual.

In some embodiments, the method further comprises the development of said predictive model using stability selection. In some embodiments, the method further comprises developing said predictive model using logistic regression. In some embodiments, the method further comprises developing said predictive model by selecting genes using stability selection with elastic-net regularized logistic regression.

The invention also pertains to a diagnostic kit for predicting progression of osteosarcoma in a subject, wherein said kit comprises at least one nucleic acid probe or oligonucleotide, which can be used in a method for measuring the level of expression of a collection of signature genes from a biological sample taken from said individual, wherein said collection of signature genes comprises at least two genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4.

Another aspect of the present invention is the use of a PPARγ inhibitor, as an antineoplastic treatment in a subject with osteosarcoma. In some embodiments, said subject is an adolescent or a young adult. In some embodiments, said subject is identified as a poor responder to chemotherapy by the method described above. In some embodiments, said inhibitor is used in combination with another antineoplastic treatments. In some embodiments, said PPARγ inhibitor is T0070907 (CAS N°313516-66-4).

## BRIEF DESCRIPTION OF THE DRAWINGS

**Figure 1: A.** Overall survival curves for G1 (pink) and G2 (blue) patients. **B.** Progression free survival curves for G1 (pink) and G2 (blue) patients. **C.** Barplot illustrating the proportion of models including clinical variables and G1/G2 stratification for the prediction of the overall survival of patients of the cohort. **D.** Principal component analysis of independent component metagene vectors. Name of the ICs for the most contributive loading variables are indicated on the plot. Bold and transparent dots show deceased and living patients respectively. Dots are colored according to G1 (pink) or G2 (blue) membership. **E.** Loadings of the most contributive ICs as estimated by partial least square discriminant analysis to stratify patients between G1 or G2.

**Figure 2:** Gene expression distribution for each of the subnetworks and their predicted biological function. Adjusted P-value computed by gene set enrichment analysis using the log2 fold change of genes from the network (G1 versus G2) to detect significant enrichment in subnetwork gene sets.

**Figure 3:** A. GISTIC analysis of the copy number alterations found significantly associated to G1 or G2 tumors. **B.** Overall survival curves of predicted G1 (pink) and G2 (blue) patients from an independent cohort of 82 osteosarcoma patients. **C.** Overall survival curves of predicted G1 (pink) and G2 (blue) patients using custom Nanostring panel on 96 samples from OS2006 cohort **D.** Barplot on the left shows proportion of G1 and G2 tumors among 39 out of 79 patients enrolled in the OS2006 trial, with tumor submitted to RNA-sequencing and which relapsed. Barplot on the right shows proportion of G1 and G2 tumors sampled at relapse among 42 patients with osteosarcoma enrolled in MAPPYACTS trial.

**Figure 4: A.** Cell viability assessment of osteosarcoma cell lines under the pressure of a PPAR antagonist (T0070907 - Blue) and two PPAR agonists (TGZ - Red or RGZ - Orange) at 48 and 72h, with concentrations of [0; 0.01; 0.1; 1; 5; 10; 17.5; 20; 25; 30; 50 or 100 μmol/L] by MTS assay. At least three independent tests were performed, with three replicates each. **B.** The protein expression of PPARγ was measured by Western Blot in osteosarcoma cell lines. Cells from normal lung tissue were used as a positive control for PPARγ expression and β-actin was used as a housekeeping gene control.

**Figure 5:** Scatter plot between each IC sample contribution and the log fold change of the CNA showing the greatest coefficient in the corresponding regression model.

**Figure 6: A.** Barplot illustrating gene selection based on their correlation between RNA-seq and Nanostring expression. Blue bars show genes correlating with p-values lower than $10^{-5}$ and selected for the Nanostring signature. The five genes in italic/bold corresponds to the housekeeping genes, chosen based on their low variance in RNA-seq, to normalized Nanostring data **B.** Progression free survival curves of predicted G1 (pink) and G2 (blue) patients using custom Nanostring panel on 96 samples from OS2006 cohort.

## DETAILED DESCRIPTION OF THE PREFERRED EMBODIMENTS

Unless otherwise indicated, the practice of the method and system disclosed herein involves conventional techniques and apparatus commonly used in molecular biology, microbiology, protein purification, protein engineering, protein and DNA sequencing, and recombinant DNA fields, which are within the skill of the art. Such techniques and apparatus are known to those of skill in the art and are described in numerous texts and reference works.

Unless defined otherwise herein, all technical and scientific terms used herein have the same meaning as commonly understood by one of ordinary skill in the art. Various scientific dictionaries that include the terms included herein are well known and available to those in the art. Although any methods and materials similar or equivalent to those described herein find use in the practice or testing of the embodiments disclosed herein, some methods and materials are described. The terms defined immediately below are more fully described by reference to the Specification as a whole. It is to be understood that this disclosure is not limited to the particular methodology, protocols, and reagents described, as these may vary, depending upon the context they are used by those of skill in the art.

In the present text, the following general definitions are used:

As used herein, the singular terms "a," "an," and "the" include the plural reference unless the context clearly indicates otherwise.

"Nucleic acid sequence," "expressed nucleic acid," or grammatical equivalents thereof used in the context of a corresponding signature gene means a nucleic acid sequence whose amount is measured as an indication of the level of expression of genes. The nucleic sequence can be a portion of a gene, a regulatory sequence, genomic DNA, cDNA, RNA including mRNA and rRNA, or others. A particular embodiment utilizes mRNA as the primary target sequence. As is outlined herein, the nucleic acid sequence can be a sequence from a sample, or a secondary target such as, for example, a product of a reaction such as a PCR amplification product (e.g., "amplicon"). A nucleic acid sequence corresponding to a signature gene can be any length, with the understanding that longer sequences are more specific. Probes are made to hybridize to nucleic acid sequences to determine the presence or absence of expression of a signature gene in a sample.

As used herein the term "comprising" means that the named elements are included, but other element (e.g., unnamed signature genes) may be added and still represent a composition or method within the scope of the claim.

As used herein, the term "signature gene" refers to a gene whose expression is correlated, either positively or negatively, with disease extent or outcome or with another predictor of disease extent or outcome. A "signature nucleic acid" is a nucleic acid comprising or corresponding to, in case of cDNA, the complete or partial sequence of a RNA transcript encoded by a signature gene, or the complement of such complete or partial sequence. A signature protein is encoded by or corresponding to a signature gene of the disclosure.

The term "relapse prediction" is used herein to refer to the prediction of the likelihood of osteosarcoma recurrence in patients with no apparent residual tumor tissue after treatment. The predictive methods of the present disclosure can be used clinically to make treatment decisions by choosing the most appropriate treatment modalities for any particular patient. The predictive methods of the present disclosure also can provide valuable tools in predicting if a patient is likely to respond favorably to a treatment regimen, such as surgical intervention, chemotherapy with a given drug or drug combination, and/or radiation therapy.

The terms "subject", "individual" or "patient" herein refer to a human subject.

"Osteosarcoma" herein designates a type of bone cancer that begins in the cells that form bones. Osteosarcoma is most often found in the long bones - more often the legs, but sometimes the arms - but it can start in any bone. In very rare instances, it occurs in soft tissue outside the bone.

The term "antineoplastic treatments" herein designate any treatment for cancer except surgery. They include chemotherapy, hormonal and biological therapies, and radiotherapy.

As used herein, "treat", "treatment" and "treating" refer to any reduction or amelioration of the progression, severity, and/or duration of cancer, particularly a solid tumor; for example in an osteosarcoma, reduction of one or more symptoms thereof that results from the administration of one or more therapies.

Other definitions will be specified below, when necessary.

According to a first aspect, the present invention pertains to an *in vitro* method for determining prognosis of osteosarcoma in an subject, comprising the steps of:

a. measuring expression levels of a collection of signature genes from a biological sample taken from said subject, wherein said collection of signature genes comprises at least two genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, GAGE12G, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4; and

b. applying the expression levels measured in step (a) to a predictive model relating expression levels of said collection of signature genes with osteosarcoma outcome; and

c. evaluating the output of said predictive model to determine prognosis of osteosarcoma in said subject.

In the above method, the predictive model can have been developed by any appropriate method known by the skilled person. Machine learning (ML) approaches have been demonstrated to be useful in medicine and can advantageously be used to build the predictive method. An example of such a method is disclosed in the experimental part below. Other examples of computational protocols for identifying and refining prognostic signatures have been described and can be used by the skilled person in the context of the present invention[66-69] (Vey et al., Cancers, 2019; Xia et al., Nature Communications, 2019; Jiang et al., Cell Systems, 2018; Liu et al, 2020; EBioMedicine).

The predictive model is advantageously developed using data collected from patients known to have osteosarcoma. The skilled person can obtain different prediction models corresponding to different clinical situations. For example, a model can be developed using data collected only from patients at diagnosis; another model can be developed using data from patients who underwent surgery of the primary tumors, *etc.*.

In some embodiments, the gene expression data may be preprocessed by normalization, background correction and/or batch effect correction. The preprocessed data may then be analyzed for differential expression of genes for stratification of patients in a good prognostic group (group 1) versus a poor prognostic group (group 2).

In some embodiments, to develop a predictive model for the prognosis of osteosarcoma, the probes included in the final model are selected from an entire set of probes using stability selection. In some embodiments, the model is developed using logistic regression, for example using elastic-net regularized logistic regression. Elastic-net regression is a high dimensional regression method that incorporates both a LASSO (L1) and a ridge regression (L2) regularization penalty. The exact mix of penalties (LASSO vs. ridge) is controlled by a parameter $\alpha$ ($\alpha = 0$ is pure ridge regression and $\alpha = 1$ is pure LASSO). The degree of regularization is controlled by the single penalty parameter. Both LASSO and ridge regression shrink the model coefficients toward zero relative to unpenalized regression but LASSO can shrink coefficients to exactly zero, thus effectively performing variable selection. LASSO alone however, tends to select randomly among correlated predictors, which the addition of the ridge penalty helps prevent. It is known that the ridge penalty shrinks the coefficients of correlated predictors towards each other while the lasso tends to pick one of them and discard the others. In some embodiments, one may use the implementation of elastic-net logistic regression in the R package *'glmnet.'*

The idea behind stability selection is to find 'stable' probes that consistently show to be predictive of recurrence across multiple data sets obtained by 'perturbing' the original data. Specifically, perturbed versions of the data are obtained by subsampling $m < n$ subjects ($n$ is the total number of subjects) without replacement. Regularized regression (or elastic-net in some embodiments) is then performed on each subsample version of the data to obtain the complete regularized path (*i.e.,* the model coefficients as a function of the regularization penalty). The effect of the LASSO penalty is to shrink the vast majority of the probe coefficients to exactly zero; the probes with non-zero coefficients (predictive) across a sizable proportion of the subsample versions of the data are deemed stable predictors.

In some embodiments, to implement stability selection with elastic net regression, one may calibrate the tuning parameter a using repeated cross-validation (e.g., using R package for a 10-fold cross-validation). The skilled person will choose the tuning parameter $\alpha$ to provide good prediction and to include as many possible features as necessary while maintaining good prediction. In some embodiments, stability selection may be implemented using different numbers of subsamples of the data, each having a portion of the total sample size (each with roughly the same proportion of cases and controls as the original), in order to identify robust predictors for the final model. In some embodiments, standardization of the gene expression levels by their standard deviation (the default in *glmnet* to place all gene features on the same scale) is not done, since differential variability of the gene expression levels may be biologically important. In some embodiments, such standardization may be performed. In some embodiments, clinical variables such as, for example, the presence of metastases at diagnosis are force included (i.e., not subject to the elastic net regularization penalty).

According to a particular embodiment illustrated in the experimental part, the predictive model leads to a stratification of patients into 2 groups: G1 of good prognosis (OS at 3 years = 100%) and G2 of poorer prognosis (OS at 3 years <70%), requiring a closer monitoring.

Patients classified in the G1 group may benefit from a treatment with an immunomodulator (e.g., mifamurtide), while patients in the G2 group may benefit from a treatment with antiangiogenesis agents like multityrosine kinase inhibitors or monoclonal antibodies, anti-osteoclasts like biphosphonates or anti-RANKL, as well as PPARgamma antagonists (as illustrated in Example 2).

Tables 4 to 8 of the experimental part below provide parameters for 5 different models which can be used to perform the above method.

In a preferred embodiment, the collection (or panel) of signature genes includes at least CCDC34 and MEAF6. Table 7 shown in the experimental part provides parameters for a model based on the expression levels of these 2 genes. Of course, these are provided as an example and not limiting. The skilled person can refine these parameters using, for example, a different cohort of patients, or adapt them to values obtained with a different technique used for measuring the expression levels of the genes. This of course holds true for all the models provided in this application.

In some embodiments, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36 or all of the 37 genes shown in Table 4 may be used in a predictive model. For illustrative purpose only, Table 4 shown in the experimental part provides parameters for a model based on the expression levels of these 37 genes.

In some embodiments, at least 5, preferably at least 7 and more preferably at least 8 genes of the list of Table 4 are included. In some embodiments, at least 10, preferably at least 12 and more preferably at least 15 genes of the list of Table 4 are included. In some embodiments, these two or more genes may be selected by their correlation with recurrence in the training data set to develop the predictive models. In some embodiments, the one or more genes may be selected by their reliability ranks. In some embodiments, the two or more genes may be selected by their predictive power rankings.

In some embodiments, the panel of signature genes comprises at least AMZ2P1, C5orf28, CCDC34, GAGE12D, MEAF6, SLC2A6, SLC7A4 and THAP9-AS1. For illustrative purpose only, Table 6 shown in the experimental part provides parameters for a model based on the expression levels of these 8 genes.

In some embodiments, the panel of signature genes comprises at least AMZ2P1, ASXL2, C11orf58, C5orf28, CCDC34, ESCO1, GAGE12D, HADHA, MEAF6, PAIP1, RALGAPA2, SLC2A6, SLC7A4, THAP9-AS1 and TIMP3. For illustrative purpose only, Table 5 shown in the experimental part provides parameters for a model based on the expression levels of these 15 genes.

In some embodiments, the panel of signature genes comprises at least AMZ2P1, C5orf28, CCDC34, ESCO1, FAM35DP, GAGE12D, HADHA, HIST2H2AA3, HIST2H2AA4, MAPK1, MEAF6, POLR2C, RALGAPA2, SLC2A6, SLC7A4, ST7L, THAP9-AS1, TIMP3 and TTPAL. For illustrative purpose only, Table 8 shown in the experimental part provides parameters for a model based on the expression levels of these 20 genes.

In some embodiments, the panel of signature genes comprises at least AMZ2P1, C5orf28, CCDC34, ESCO1, GAGE12D, HADHA, MEAF6, RALGAPA2, SLC2A6, SLC7A4, THAP9-AS1 and TIMP3. These 12 genes are present in the models shown in both Table 5 and Table 8.

The method of the invention, in any of its embodiments described above, can further comprise combining the gene expression levels of said signature genes with one or more other parameters to predict progression of osteosarcoma in said subject. Non-limitative examples of such clinical parameters which can be combined to the expression levels of the selected genes include the presence and amount of metastases at diagnosis, the size, height and/or stage of the tumor at diagnosis, drugs administered to the patient, age, and any combinations thereof.

In some embodiments, based on the expression levels for the set of probes determined by stability selection and the clinical variables, the predictive model is obtained by fitting logistic model using elastic-net regularized logistic regression. Glmnet solves the following problem

$$\min_{(\beta_0,\beta)\in\mathbb{R}^{p+1}} -\left[\frac{1}{N}\sum_{i=1}^{N} y_i \cdot (\beta_0 + x_i^T\beta) - \log(1 + e^{(\beta_0 + x_i^T\beta)})\right] + \lambda\left[(1-\alpha)||\beta||_2^2/2 + \alpha||\beta||_1\right]$$

over a grid of values of λ covering the entire range. The tuning parameter λ controls the overall strength of the penalty.

In some embodiments, instead of selecting a subset of genes from the panel disclosed in Table 4, a model may weight the genes differently in the logistic regression. In some embodiments, determining the prognosis of osteosarcoma for an individual involves applying expression levels of the collection of signature genes to the predictive model, which involves weighting said expression levels according to stability rankings of the collection of signature genes. In some embodiments, the method involves weighting expression levels according to predictive power rankings of the collection of signature genes.

The logistic regression model above expresses the specific way the expression levels and the clinical variables are combined to obtain a score for each individual. In some embodiments, expression levels are weighted in the elastic-net regularized logistic regression. In some embodiments, expression levels are weighted in using LASSO. The weighting here does not refer to the model coefficients (which can be thought of as weights for the expression levels and clinical variables), but rather to an additional mechanism for differentially accounting for variable importance in the logistic regression procedure. In this regard, alternative embodiments consider unweighted logistic regression, *i.e.,* treating all genes equally, and weighted logistic regression, weighting by the stability selection frequencies.

In some embodiments, various clinical variables (e.g., drugs administered to the patient, age, tumour height and stage at diagnosis, presence of absence of metastasis at diagnosis) will be included in the same logistic model along with the signature genes. Coefficients will be defined for each variable (gene expression and clinical values). This logistic regression model will provide a probability of having a clinical recurrence given the provided gene expression scores and clinical variables. This probability will be a number between 0-1, and it will indicate for each given patient the prognosis of the disease.

In some embodiments, in addition to identifying the coefficients of the predictive model, the disclosure identifies the most useful specificity and sensitivity a user wishes to have for a specific risk probability. Based on the desired specificity and sensitivity levels, the method will report the risk status of each patient. For example, the skilled person may find that given the specificity and sensitivity of our model, a patient with 45% chance of being classified in the good prognosis responders group (group 1) might be better of being classified in the poor progbosis responders group (group 2) rather than group 1 or vice versa. In other words, more user-friendly criteria can be chosen based on more detailed analysis in further datasets to determine the most practical interpretation of the risk probability depending on how much clinicians want to risk having a false positive or a false negative.

Individuals suspected of having any of a variety of bone cancers, such as osteosarcoma, osteocarcinoma or cancer of the bones joints and soft tissue as well as pediatric sarcomas, can be evaluated using a method of the disclosure. Exemplary cancers that can be evaluated using a method of the disclosure include, but are not limited to osteosarcoma, pediatric sarcoma a well as adult and pediatric relapsing cancers in bone tissue.

Exemplary clinical outcomes that can be determined from a model of the disclosure include, for example, response to a particular course of therapy such as surgical removal of a tumor, radiation, or chemotherapy.

The skilled person will appreciate that patient tissue samples containing osteosarcoma cells may be used in the methods of the present disclosure including, but not limited to those aimed at determining the prognosis of the disease. In these embodiments, the level of expression of the signature gene can be assessed by assessing the amount, e.g. absolute amount or concentration, of a signature gene product, e.g., protein and RNA transcript encoded by the signature gene and fragments of the protein and RNA transcript) in a sample obtained from a patient. The sample can, of course, be subjected to a variety of well-known post-collection preparative and storage techniques (e.g. fixation, storage, freezing, lysis, homogenization, DNA or RNA extraction, ultrafiltration, concentration, evaporation, centrifugation, etc.) prior to assessing the amount of the signature gene product in the sample.

Tissue samples useful for preparing a model for determining the prognosis of osteosarcoma include, for example, paraffin and polymer embedded samples, ethanol embedded samples and/or formalin and formaldehyde embedded tissues, although any suitable sample may be used. In general, nucleic acids isolated from archived samples can be highly degraded and the quality of nucleic preparation can depend on several factors, including the sample shelf life, fixation technique and isolation method.

If required, a nucleic acid sample having the signature gene sequence(s) are prepared using known techniques. For example, the sample can be treated to lyse the cells, using known lysis buffers, sonication, electroporation, etc., with purification and amplification as outlined below occurring as needed, as will be appreciated by those in the art. In addition, the reactions can be accomplished in a variety of ways, as will be appreciated by those in the art. Components of the reaction may be added simultaneously, or sequentially, in any order, with preferred embodiments outlined below. In addition, the reaction can include a variety of other reagents which can be useful in the assays. These include reagents like salts, buffers, neutral proteins, e.g. albumin, detergents, etc., which may be used to facilitate optimal hybridization and detection, and/or reduce non-specific or background interactions. Also reagents that otherwise improve the efficiency of the assay, such as protease inhibitors, nuclease inhibitors, anti-microbial agents, etc., can be used, depending on the sample preparation methods and purity.

In some embodiments, biological samples in addition to or instead of osteosarcoma tissue may be used to determine the expression levels of the signature genes. In some embodiments, the suitable biological samples include, but are not limited to, circulating tumor cells isolated from the blood, urine of the patients or other body fluids, exosomes, and circulating tumor nucleic acids.

According to a particular embodiment, the expression levels of said collection of signature genes are measured at diagnosis.

According to another particular embodiment, the expression levels of said collection of signature genes are measured at relapse.

In some embodiments, the gene expression levels of the signature genes may be measured multiple times. In some embodiments, the dynamics of the expression levels may be used in combination of the signature genes expression levels to better predict the clinical outcome. One skilled in the art understands various approaches may be used to combine the effects of the levels and the dynamics of the signature genes' expression to determine the prognosis of osteosarcoma.

The methods of the disclosure depend on the detection of differentially expressed genes for expression profiling across heterogeneous tissues. Thus, the methods depend on profiling genes whose expression in certain tissues is activated to a higher or lower level in an individual afflicted with a condition, for example, cancer, such as osteosarcoma, relative to its expression in a non-cancerous tissues or in a control subject. Gene expression can be activated to a higher or lower level at different stages of the same conditions and a differentially expressed gene can be either activated or inhibited at the nucleic acid level or protein level.

Differential signature gene expression can be identified, or confirmed using methods known in the art such as qRT-PCR (quantitative reverse-transcription polymerase chain reaction) and microarray analysis. In particular embodiments, differential signature gene expression can be identified, or confirmed using microarray techniques or any similar technique (e.g., NanoString technique). Thus, the signature genes can be measured in either fresh or paraffin-embedded tumor tissue, using microarray technology. In this method, polynucleotide sequences of interest are plated, or arrayed, on a microchip substrate. The arrayed sequences are then hybridized with specific DNA probes from cells or tissues of interest.

The expression level of a signature gene in a tissue sample can be determined by contacting nucleic acid molecules derived from the tissue sample with a set of probes under conditions where perfectly complementary probes form a hybridization complex with the nucleic acid sequences corresponding to the signature genes, each of the probes including at least two universal priming sites and a signature gene target-specific sequence; amplifying the probes forming the hybridization complexes to produce amplicons; and detecting the amplicons, wherein the detection of the amplicons indicates the presence of the nucleic acid sequences corresponding to the signature gene in the tissue sample; and determining the expression level of the signature gene.

The expression level of nucleic acid sequences corresponding to a set of signature genes in a tissue sample can be determined by contacting nucleic acid molecules derived from the tissue sample with a set of probes under conditions where complementary probes form a hybridization complex with the signature gene-specific nucleic acid sequences, each of the probes including at least two universal priming sites and a signature gene-specific nucleic acid sequence; amplifying the probes forming the hybridization complexes to produce amplicons; detecting the amplicons, wherein the detection of the amplicons indicates the presence of the nucleic acid sequences corresponding to the set of signature genes in the tissue sample; and determining the expression level of the target sequences, wherein the expression of at least two, at least three, at least five signature gene-specific sequences is detected.

The present invention also pertains to a collection of isolated probes specific for osteosarcoma signature genes comprising at least two genes selected from the group consisting of AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, GAGE12G, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4. In particular, the invention provides, for each of the collection of signature genes mentioned above, an appropriate collection of probes. Examples of such probes are provided in Table 3 below.

The invention includes compositions, kits, and methods for determining the prognosis of osteosarcoma for an individual from which a sample is obtained. A kit is any manufacture (e.g. a package or container) including at least one reagent, *e.g.* a probe or a collection of probes as above-described (e.g., nucleic acid probes of SEQ ID NO: 1-41 or a subset thereof), for specifically detecting the expression of a gene signature as described herein.

As shown in Example 2 below, the inventors demonstrated that a PPARγ antagonist decreases the cell viability in 7 human osteosarcoma lines. This is a proof of principle that inhibitors of the PPARγ pathway may constitute a breakthrough innovation in the armamentarium for treating osteosarcoma.

In another of its aspects, the present invention thus pertains to the use of a PPARγ inhibitor as an antineoplastic treatment in a subject with osteosarcoma, especially for treating an adolescent or a young adult.

Examples of PPARγ inhibitors which can be used according to the present invention include T0070907 (CAS N°313516-66-4), BADGE, FH 535, GW 9662, SR 16832, SR 202 and combinations thereof.

According to a particular embodiment, a PPARγ inhibitor is used for treating a patient who has been identified as a poor responder to chemotherapy by a method of the invention, as above-described.

In a particular embodiment, the PPARγ inhibitor can be combined to another treatment, such as a treatment with antiangiogenesis agents (e.g., multityrosine kinase inhibitors and monoclonal antibodies), anti-osteoclasts (e.g., biphosphonates and anti-RANKL), *etc.*

Other characteristics of the invention will also become apparent in the course of the description which follows of the biological assays which have been performed in the framework of the invention and which provide it with the required experimental support, without limiting its scope.

## EXAMPLES

The inventors reasoned that in osteosarcoma, gene expression at RNA level, in addition to giving access to the TME composition, should better describe osteosarcoma tumors as a final read-out of the epigenetic and transcriptional modulation of a highly rearranged genetic landscape than phenotypic features.

In order to test this hypothesis, we performed RNA-sequencing (RNA-seq) of 79 primary osteosarcoma tumors sampled at diagnosis from patients accrued in and homogenously treated by the first line OS2006 trial (NCT00470223). These samples recapitulate the main clinical feature distribution of the whole cohort (Table 1). To study the transcriptome landscape of both osteosarcoma and TME cells, we first dissociated their respective transcriptome programs. To do so, we decomposed RNA gene expression matrix by independent component analysis (ICA, BIODICA implementation)[23] with ICASSO stabilization procedure[23] into 50 independent components (ICs), also called gene modules.

**Table 1: Patient primary tumor biopsy sample cohort explored by RNA-seq analysis in OS2006 trial.** Patients and tumor characteristics, treatment and outcome of the 79 patients with RNA-seq analysis in the first-line phase-III therapeutic OS2006 trial.

| | | OS2006 | OS2006 RNA-seq | OS2006 | OS2006 RNA-seq |
|---|---|---|---|---|---|
| | Age (median) | 15,3 | 15,45 | - | - |
| Sex | Male Female | 338 | 46 | 0,550 | 0,575 |
| | | 276 | 34 | 0,450 | 0,425 |
| Puberty | Prepuberty Intrapuberty postpuberty | 36 | 2 | 0,059 | 0,025 |
| | | 137 | 18 | 0,223 | 0,225 |
| | | 131 | 20 | 0,213 | 0,250 |
| | | 310 | 40 | 0,505 | 0,500 |
| Future Relapse | Yes No | 251 | 29 | 0,409 | 0,363 |
| | | 363 | 51 | 0,591 | 0,638 |
| Metastases at diagnostic | - Localised Osteosarcoma Possible Metastases Definite metastases | 18 | 0 | 0,029 | 0,000 |
| | | 382 | 49 | 0,622 | 0,613 |
| | | 112 | 15 | 0,182 | 0,188 |
| | | 102 | 16 | 0,166 | 0,200 |
| Fisrt instance Chimiotherapy | - API-AI MTX | 16 | 1 | 0,026 | 0,013 |
| | | 111 | 10 | 0,181 | 0,125 |
| | | 487 | 69 | 0,793 | 0,863 |

(continued)

| | | OS2006 | OS2006 RNA-seq | OS2006 | OS2006 RNA-seq |
|---|---|---|---|---|---|
| Histologic response | - Good responder Poor responder | 51 | 2 | 0,086 | 0,025 |
| | | 335 | 58 | 0,566 | 0,734 |
| | | 206 | 19 | 0,348 | 0,241 |
| Status | Alive Dead | 429 | 58 | 0,699 | 0,725 |
| | | 185 | 22 | 0,301 | 0,275 |

**Example 1: Immune infiltrate and tumor microenvironment transcriptional programs stratify pediatric osteosarcoma into prognostic groups at diagnosis**

**Materials and Methods**

***Population and Materials***

Biological samples were prospectively collected for patients (up to 50 years) registered into the therapeutic approved French OS2006/sarcoma09 trial (NCT00470223)[6]. This study was carried out in accordance with the ethical principles of the Declaration of Helsinki and with Good Clinical Practice guidelines. A specific informed consent for blood and tumor samples was obtained from patients or their parents/guardians if patients were under 18 years of age upon enrolment. The information given to the patients, the written consent used, the collection of samples and the research project were approved by an independent ethic committee and institutional review boards. As part of the ancillary biological studies, RNA-seq, CGH array and WES were performed at Gustave Roussy Cancer Campus.

Between 2009 and 2014, 79 frozen osteosarcoma biopsy samples at diagnosis were collected and analyzed by RNA-seq. The clinical and survival characteristics of the 79 patients (Table 1) were comparable to the whole osteosarcoma population registered in the OS2006 trial. The sex ratio M/F was 0.55 and the median age of 15.43 years (4.71-36.83). Primary tumors were located in the limb (94.9%) and 20.2% had metastases at diagnosis. Chemotherapy was administered as per OS2006 trial; either MTX-etoposide/ifosfamide (M-EI) regimen in 86% of the patients or adriamycin/platinum/ifosfamide (API-AI) regimen for 12.6% of patients, and 34.1% received zoledronic acid according to the randomization arm. 77 patients had surgery of the primary tumor and poor histological response was observed in 24 % of the patients. 29 patients relapsed with a median delay of 1.55 years (range 0.09.-3.62). 22 patients died with a median delay of 2.25 years (range 0.07.-6.9). The median follow-up was of 4.8 years. Overall, the 3-year PFS and OS were of 65.82% and 82.27%, respectively.

***Nucleic acids extraction***

DNA and RNA were isolated using AllPrep DNA/RNA mini kit (Qiagen, Courtaboeuf, France) according to manufacturer's instructions. Quantification/qualification were performed using Nanodrop 2000 spectrophotometer (Thermo Fisher Scientific, Illkirch, France) and Bioanalyzer DNA 7500 (Agilent Technologies, Les Ulis, France).

***RNA sequencing***

RNA sequencing libraries were prepared with TrueSeq Stranded mRNA kit following recommendations: the key steps consist of PolyA mRNA capture with oligo dT beads using 1μg total RNA, fragmentation to approximately 400pb, DNA double strand synthesis, and ligation of Illumina adaptors amplification of the library by PCR for sequencing. Libraries sequencing was performed using Illumina sequencers (NextSeq 500 or Hiseq 2000/2500/4000) in 75 bp paired-end mode in both techniques and data sequencing were processed by bioinformatics analyses. For the optimized detection of potential fusion transcripts by RNA-seq an in-house designed metacaller approach was used.

***Gene expression analysis in RNA-seq***

Quality of stranded pair-ended RNA-seq libraries was evaluated with fastqc (a quality control tool for high throughput sequence data available on the Babraham bioinformatics pages of the website of the Babraham institute). Reads were mapped with RSEM using GRCh37 ENSEMBL mRNA dataset as reference sequences.

ICA was performed using BIODICA, one of the most performant implementations of fastICA including icasso stability analysis. Genes represented by less than 100 reads were filtered out from the ICA and gene expression matrix was log scaled and scaled by genes before decomposition in 50 components. Pearson correlations between ICs, to assess inter IC relationship, were calculated from the metagenes matrix. For each IC we defined contributive genes, as genes within 3 standard deviations of the mean of the corresponding IC metagene vector. For each component, gene set enrichment was performed using msigdb for positional enrichment and g:Profiler for functional enrichment. For each component, only the most significant enrichment detected among the GO, REACTOM E or TF categories has been reported. For positional enrichment, only the cytobands with a minimum FDR of $1e^{-4}$ have been reported. When several cytobands from the same chromosomal arm were found enriched only the less significant p-values have been reported in Table 2. From the gene expression matrix, we selected only the contributive genes, within 2.5 standard deviations of the mean of the corresponding IC metagenes vector, to perform a network inference using Pearson distance to calculate edge lengths. Edges with distance above 0.25 were discarded. Network and subnetwork illustrations and analyses have been produced with Cytoscape v3.6.1. We used the REACTOME FI viz plugin to detect clusters in the network with the spectral partition based network clustering algorithm and estimate for each cluster their functional enrichments in GO biological process terms.

Hierarchical classification of metagene matrix and corresponding heatmap was generated with pheatmap R package using Pearson distance and Ward's construction method. Pls-da function from the mixOmics R package was used to select the most contributive independent components to the G1/G2 patient classification.

Kaplan-Meier OS and PFS curves were generated using the survminer and survival R packages. Log-rank test were used to compare survival distribution in-between groups and to provide corresponding p-values.

**Table 2: Functionnal annotation of the independent components by functional enrichment based on MSigDB and g:Profiler databases**

| | Functional annotations | Chromosomal regions | Adj. p. values |
|---|---|---|---|
| IC6 | | 6p21 | 7e-180 |
| IC22 | | **19p13** | 0 |
| IC24 | | 15q22-25 | <1$^{e}$-19 |
| IC34 | | 4p15/p16 | <1$^{e}$-24 |
| IC39 | | **Xp11** | 2 .8$^{e}$-108 |
| IC14 | | **9p13/p21** | <1.e-23 |
| IC35 | | 6q14-16/q21 13q32-34 | <1$^{e}$-8 |
| IC20 | Cell cycle (GO :BP) | | 1.6$^{e}$-94 |
| IC44 | Ossification (GO :BP) | | 5.4$^{e}$-9 |
| IC5 | | **12p11-13** | <1$^{e}$-23 |
| IC3 | | **14q21-24/q32** | <1$^{e}$-32 |
| IC29 | | Xq22/q24/q26/q28 | <1$^{e}$-24 |
| IC43 | Translation/Ribosome | | <1-e16 |
| IC11 | | **7q21-22/q31-36** | <1$^{e}$-13 |
| IC38 | | 10q11-15/q21-24 | <1$^{e}$-5 |
| IC1 | Muscle system | | 1.4$^{e}$-58 |
| IC2 | Y-linked inheritance | Yp11/q11 | 1.5$^{e}$-4/3.1$^{e}$-24 |
| IC21 | | **17q11/q12/q21** | <1$^{e}$-28 |
| IC4 | | **8p11-12/p21-23** | <1$^{e}$-9 |
| IC10 | | **17p11-12** | <1$^{e}$-20 |
| IC40 | | 3q13/q21-29 | <1$^{e}$-6 |
| IC37 | | **12q23-24** | <1$^{e}$-24 |
| IC48 | Somatodendritic (GO:CC) | | 1.8$^{e}$-3 |

(continued)

| | Functional annotations | Chromosomal regions | Adj. p. values |
|---|---|---|---|
| IC49 | EGR1 (TF) | | 2.6$^{e}$-4 |
| IC8 | | **9q21-22/q31-34** | <1$^{e}$-12 |
| IC30 | | **11a12-13/q21-25** | <1$^{e}$-5 |
| IC16 | | **7p13-15/21-22** | <1$^{e}$-5 |
| IC36 | | | |
| IC45 | | 1p34-36 | <1$^{e}$-30 |
| IC7 | | **1 q41-42/q32** | <1$^{e}$-23 |
| IC31 | | | |
| IC33 | | **22q11-13** | <1$^{e}$-80 |
| IC19 | | 6p21-25 | <1$^{e}$-7 |
| IC23 | | 8q12-13/q21-24 | <1$^{e}$-5 |
| IC28 | | **15q14-15/q21-22** | <1$^{e}$-16 |
| IC27 | | **5q11-15/q22-23/q31** | <1$^{e}$-8 |
| IC46 | | | |
| IC42 | | 4p31-34 3p13/p21 | <1$^{e}$-6 |
| IC15 | | **17p13** | 3.1$^{e}$-174 |
| IC50 | | | |
| IC13 | Angiogenesis (GO:BP) | | 6.8$^{e}$-31 |
| IC25 | Bone resorbption (GO:BP) | | 2.5$^{e}$-14 |
| IC17 | Transcription (GO:MF) | | |
| IC41 | Immune response (GO:BP) | | 2.8$^{e}$-133 |
| IC26 | Cancer Testis Antigen | | |
| IC47 | | | |
| IC9 | Neutrophil degranulation | | 6.5$^{e}$-28 |
| IC12 | | **16q21-24** | <1$^{e}$-9 |
| IC18 | | **19q13** | 3$^{e}$-306 |
| IC32 | | **16p11-13** | <1$^{e}$-43 |

Differential mRNA expression was estimated with DESeq2 R package from raw read count table.

A gaussian regression model using the glmnet R package (parameters: type.measure="mse", alpha=0.8, family="gaussian") was used to select CNAs (inferred from CGH array in pair with RNA-seq samples) modeling the best each IC. Likewise, we detected dependencies between ICs corresponding to large chromosomal transcriptional modulation and copy number alterations from the same chromosomal region. Chromosomal region written in bold letters in Table 2 corresponds to the region, enriched in ICs, overlapping with the CNA contributing the most to the model, which suggest a dose-dependent relationship. Figure 5 illustrates those cases by the scatter plot of IC signal vs copy number log fold change of the copy number alteration with the highest coefficient in the regression model.

A logistic regression model using the glmnet R package was used to define a minimum gene signature able to discriminate G1 from G2 RNA-sequencing libraries (parameters: type.measure="mse", alpha=0.35, family="binomial"). Cross-validation strategy was used to select the best lambda. We then predicted with this signature G1 and G2 tumors from the 82 osteosarcoma RNA-seq dataset generated by the TARGET consortium as well as compared the signature in 42 relapsed osteosarcoma samples in the MAPPYACTS trial (NCT02613962). For TARGET and MAPPYACTs, gene expression was scaled by the mean and the variance of the gene expression in our 79 samples. For TARGET samples, Kaplan-Meier OS and PFS curves were generated using the survminer and survival R packages. Log-rank test were used to compare survival distribution in-between groups and to provide corresponding p-values.

***Oligonucleotide Array Comparative Genomic Hybridization (aCGH) assay.***

In all experiments, sex-matched normal DNA from a pooled human female or male (Promega, Madison, WI, USA) was used as a reference. Oligonucleotide aCGH processing was performed as detailed in the manufacturer's protocol (version 7.5; http://www.agilent.com). Equal amounts (500 ng) of tumor and normal DNAs were fragmented with AluI and RsaI (Fermentas, Euromedex, France). The fragmented DNAs were labelled with cyanine Cy3-deoxyuridine triphosphate (dUTP) or Cy5-dUTP. Hybridization was carried out on SurePrint G3 Human CGH Microarray 4x180K (Agilent Technologies, Santa Clara, CA, USA) arrays for 24 hours at 65°C in a rotating oven (Robbins Scientific, Mountain View, CA) at 20rpm. The hybridization was followed by appropriate washing steps.

***Oligonucleotide aCGH mixed (joint + individual) preprocessing.***

Scanning of glass microarrays was performed with an Agilent G2505C DNA Microarray scanner at 100% PMT with 3 μm resolution at 20°C in low ozone concentration environment. Data were extracted from scanned TIFF images using the Feature Extraction software (v11.5.1.1, Agilent), along with protocol CGH_1105_Oct12. All further data treatments were performed under the R statistical environment in v3.4 (http://cran.r-project.org). Acquired raw intensities were transformed to log2(test/ref). Joint normalization of the whole cohort of raw CGH profiles was performed using the 'cghseg' package (v1.0.2.1) using default parameters: a common "wave-effect" track was computed, then subtracted to all individual profiles through a lowess regression. As a second step, an individual normalization step was performed for each profile by subtracting pre-computed GC-content tracks through a lowess regression. Joint segmentation of the whole cohort of normalized CGH profiles was performed using a penalized least-square regression implemented in the 'copynumber' package (v1.16.0). To set a unique value to the gamma (penalty) parameter for the whole cohort, optimal gamma value was computed for each individual profile using 100 cross-validation folds, and the median of optimal gammas was chosen (median = 18, sd = 4.2, range = 11:41). Joint segmentation resulted into 1604 segments. Individual centering of profiles was performed using an in-house method selecting the most-centered mode in the distribution density of probes' log2(ratio) values. No calling of aberrations was performed.

***Oligonucleotide aCGH analysis.***

All genomic coordinates were established on the UCSC human genome build hg19. Hierarchical clustering of samples was performed under R on segmented data, using [[Euclidean / Pearson / Spearman]] distances and Ward's construction method. Clustering of samples based on non-negative matrix factorization and spherical k-means were performed using the 'NMF' (v0.20.6) and 'skmeans' (v0.2.10) packages, respectively. Minimum common regions (MCR) analysis was performed using GISTIC2 (v2.0.22). Differential analyses were performed using non-parametric statistical tests (Wilcoxon for 2-classes, Kruskal-Wallis for N-classes), and all p-values were FDR-adjusted using the Benjamini-Hochberg method.

***Multivariate proportional hazards model***

To model the association between the overall survival and the major clinical factors (sex, histological response, metastatic status , tumor size, treatment, chemotherapy pubertal status) and the G1/G2 signature, we considered stability selection with boosting using Cox model (Hofner et al., 2015: Controlling false discoveries in high-dimensional situations: boosting with stability selection; BMC Bioinformatics volume 16, Article number: 144).

The selection frequencies to these predictors were computed from 1,000 bootstrap samples. We fixed the number $q$ of selected variables per boosting run to 2, and the threshold to define stable variable $n_{th}$ to 0.9 (which can be relaxed, increasing the risk of false positive predictor selection). The per-family error rate (PFER), corresponding to the expectation of the maximum number of false positive selected predictors, and the significance level were computed from the two previously defined quantities ($q$ and $\pi_{th}$) from the definition provided by Meinshausen and Bühlmann (Meinshausen and Bühlmann (2010); Stability selection; Royal Statistical Society 1369-7412/10/72417J. R. Statist. Soc. B (2010)72, Part 4, pp. 417-473). These analyses were performed using the *mboost* R package.

***Targeted RNA expression analysis***

A direct gene expression analysis was conducted with a Nanostring standard custom approach and following the supplier recommendations. A specific panel of 41 probes of interest including five housekeeping genes (CNOT1, EIF4G2, SF1, SLC39A1, and SURF4) was designed according to Nanostring. (Table 3 List of targets). RNA integrity was measured with a Fragment Analyzer system (RNA concentration, RNA Quality Number, Percentage of RNA fragments > 300nt), RNA concentrations and purity were controlled with a Nanodrop ND8000.

**Table 3: List of targets**

| **SEQ ID No :** | Target name | Accession | Position |
|---|---|---|---|
| 1 | AMZ2P1 | NR_026903.1 | 3178-3277 |
| 2 | ASXL2 | NM_018263.4 | 3361-3460 |
| 3 | BIRC6 | NM_016252.3 | 1213-1312 |
| 4 | C11orf58 | NM_014267.3 | 526-625 |
| 5 | C1orf53 | NM_001024594.2 | 373-472 |
| 6 | CCDC34 iso 1 | NM_030771.2 | 865-964 |
| 7 | CCDC34 iso 2 | NM_080654.2 | 886-985 |
| 8 | CNOT1 | NM_206999.1 | 406-505 |
| 9 | DCUN1D2 | NM_001014283.1 | 1211-1310 |
| 10 | DNMT3A | NM_022552.4 | 2057-2156 |
| 11 | ECI1 | NM_001919.3 | 837-936 |
| 12 | EIF3M | NM_006360.4 | 649-748 |
| 13 | EIF4G2 | NM_001042559.2 | 1536-1635 |
| 14 | ESCO1 | NM_052911.2 | 1531-1630 |
| 15 | GAGE12D | NM_001127199.1 | 150-249 |
| 16 | HIST2H2AA3 | NM_003516.2 | 138-237 |
| 17 | HADHA | NM_000182.4 | 1401-1500 |
| 18 | MAPK1 | NM_138957.2 | 431-530 |
| 19 | MEAF6 | NR_073091.1 | 177-276 |
| 20 | MYO1B | NM_001130158.1 | 2476-2575 |
| 21 | MYOM3 | NM_152372.3 | 3251-3350 |
| 22 | NDP | NM_000266.2 | 506-605 |
| 23 | PAIP1 | NM_006451.4 | 786-885 |
| 24 | PCID2 | NM_001127202.1 | 1076-1175 |
| 25 | PEG10 | NM_001040152.1 | 5001-5100 |
| 26 | PEX26 | NM_001199319.1 | 1247-1346 |
| 27 | POLR2C | NM_032940.2 | 1036-1135 |
| 28 | RALGAPA2 | NM_020343.2 | 2461-2560 |
| 29 | SF1 | NM_004630.2 | 967-1066 |
| 30 | FAM35DP | NR_027634.1 | 1737-1836 |
| 31 | SLC2A6 | NM_001145099.1 | 175-274 |
| 32 | SLC39A1 | NM_001271957.1 | 1545-1644 |
| 33 | SLC7A4 | NM_004173.2 | 1741-1840 |
| 34 | ST7L | NM_001308264.1 | 649-748 |
| 35 | SURF4 | NM_033161.2 | 2426-2525 |

(continued)

| SEQ ID No : | Target name | Accession | Position |
|---|---|---|---|
| 36 | THAP9-AS1 | NR_034076.1 | 229-328 |
| 37 | TIMP3 | NM_000362.4 | 1641-1740 |
| 38 | C5orf28 | NM_022483.4 | 147-246 |
| 39 | TTPAL | NM_001039199.1 | 1576-1675 |
| 40 | UBXN4 | NM_014607.3 | 344-443 |
| 41 | WNT4 | NM_030761.3 | 626-725 |

Due to the number of targets (<400), 100ng of total RNA were hybridized to Nanostring probes. Our cohort composed of 176 samples was splitted in two batches of hybridizations, including a No Template Control (NTC, water) sample and a Universal RNA sample (Agilent Technologies, P/N: 740000) per batch. Before hybridization, Nanostring positive and negative controls were added to samples as spike in controls. Probes and mRNA were hybridized 16h at 65°C prior processing on the NanoString nCounter preparation station to remove excess of probes and immobilize biotinylated hybrids on a cartridge coated with streptavidin. Cartridges were scanned at a maximum scan resolution (555 fields of view (FOV)) on the nCounter Digital Analyzer (NanoString Technologies) to count individual fluorescent barcodes and quantify RNA molecules. The Nanostring nSolver 4.0 software was used to control raw data, and to normalize data. Imaging QC criteria was higher than 93% (threshold: 75% of FOV). Binding density was monitored as Positive and Negative controls signals. Counts obtained for each target in NTC samples (1-9 counts) were deduced to corresponding target in samples of interest. Geometric average of Housekeeping gene signals was used to normalize RNA content. Comparison of Universal RNA were in the platform agreements (r2: 0.98792). Out of 176 samples, three samples with low performances were rejected from the primary analysis: one sample has a low binding density (lower threshold: 0.1; EX148_ARN010: 0.09) and two samples have a high mRNA content normalization factor (threshold: 20; EX148_ARN071: 25.7 and EX148_ARN165: 870.6).

***G1/G2 strata prediction from targeted RNA expression panel***

To detect suspicious discrepancies, we first compared the Nanostring and RNA-seq estimation of the RNA expression of the 35 genes as well as the 5 housekeeping genes. Likewise, we filtered out 7 genes with a p-value of correlation (pearson method) higher than $10^{-5}$ (Figure not shown, Fig 6A). From the 166 samples, 70 samples, already analyzed by RNA-seq and therefore classified G1 or G2, were chosen as training set to retrain our logistic model with elastic net regularization using glmnet on the 28 targeted RNA expression panel. Alpha was chosen to 0.8 and lambda by a cross validation strategy. Then we predicted the G1/G2 strata on the 96 remaining samples and Kaplan-Meier OS and PFS curves were generated using the survminer and survival R packages. Log-rank test were used to compare survival distribution in-between groups and to provide corresponding p-values.

## Results

### 1.1. Independent components recapitulate biological functions and their interations

We first tested whether specific ICs were separately correlated to clinical variables. Apart from IC2, associated with sex and obvious gene enrichment from Y chromosome (Figure not shown), no other ICs were significantly associated with clinical items after p-value adjustment.

We next characterized ICs by functional enrichment analysis using the most contributive genes of each IC. We observed 90% of the IC are associated significantly to either large chromosomal region over transcriptional modulation with msigdb database or specific cellular/molecular functions with g:Profiler (Table 2). Several ICs appeared specific to tumor clones with altered gene expression involving statistically significant large chromosomic regions. Some of these regions recapitulate known CNVs observed in osteosarcomas, as confirmed by a comparison to 70 CGH arrays produced in pair with our RNA-seq samples (Table 2, Fig 5). Contributive genes from the remaining 10 ICs recapitulate the transcriptional program of diverse TME cells or their interaction with tumor cells. We identified osteosarcoma functional gene modules belonging to bone microenvironment (osteoclasts/bone resorption IC25; osteoblast/ossification IC44), angiogenesis (IC13), immune response (IC41), but also neuron projection (IC48) and muscle (IC1). Pearson correlation study of the components revealed a proximity between specific ICs with two main groups of components (Figure not shown). Such IC clustering shows that osteosarcoma tumors share two specific IC patterns and also confirms strong relationship across TME, cancer cells and large chromosomal region modulations. To decipher these potential functional interactions, we inferred a co-expression network using only the genes contributing significantly to ICs (>3 standard deviation from the mean). Genes belonging to the same ICs demonstrate strong interconnection in the network, confirming the existence of a continuum between ICs latent variables and real measured gene expression (Figure not shown). In most cases, we identified matches between the ICs and functional subnetwork annotations performed with REACTOME FI viz (Figure not shown), confirming the potency of ICA to decompose gene expression matrix from heterogeneous tumor into functionally relevant gene modules. In addition, annotation of the co-expression network helped us to classify genes from ICs into more precise subnetworks. IC41, initially flagged as general immune response, has been subdivided into "adaptive immune response/neutrophil degranulation", "Type 1 interferon response" and "adaptive immune response". IC9 has been subdivided into "neutrophil degranulation/defensin" and "adipogenesis/peroxisome proliferator-activated receptor (PPAR) signaling pathway". At a larger scale, the network is structured around several hubs of highly connected ICs/subnetworks, probably mirroring the active crosstalk between TME and tumor cells.

### 1.2. Stable independent components stratify patients by prognosis

We questioned whether ICs/subnetwork interconnections reflecting the TME composition were linked to clinical variables. We categorized tumors by hierarchical classification using stable ICs (Stability index>0.5) reflecting clinical annotations (Figure not shown). This unsupervised analysis defined two groups of tumors associated to different average "living status" and discriminating the cohort into 35 low risk tumors, coined G1 (8.5% Death Rate, DR), from a higher risk group G2 (43.1% DR; Figure not shown). We confirmed the significant different overall survival (OS), estimated by the Kaplan-Meier method, between G1/G2 groups using log-rank test (p-value=0.00042; Fig 1A). With a median follow-up of 4.8 years, the 3y-OS was of 100% and 67.8% for G1 and G2 respectively. Difference was also observed for progression-free survival (PFS) although less significant (p-value=0.042; Fig 1B). We next challenged the prognostic value of our stratification with a multivariate proportional hazard model including known osteosarcoma prognostic factors. Model tested in this stability analysis identified the G1/G2 stratification at diagnosis as the most contributive factor to OS (with 68.5% inclusion frequency; Fig 1C). To refine our analysis, we selected ICs contributing the most to the prognostic G1/G2 stratification by their projection in the first two principal component spaces (Fig 1D). As confirmed by a supervised partial least square discriminant analysis (Fig 1E), IC39 best represents the G1/G2 stratification but several other ICs participate to the distinction between both groups (Fig 1D, 1E). First principal component (PCA1) confirmed high association of several ICs to the prognosis, when the second component (PCA2) has not been significantly associated to any clinical item. Therefore, although PCA2 might be associated to interesting state of tumor cells (IC48: neuroprojection, IC49: EGR1 regulated genes, IC50: KIT and YES1 expression, IC42: E2F1 regulated genes), we further focused our work on the PCA1 functions related to survival. These observations support our hypothesis that the complex traits related to our stratification and patient prognosis emerged from the interaction of several gene modules.

### 1.3. Stratifying ICs are related to specific biological functions

To understand at the biological level this hidden complex tumor trait detectable at diagnosis, we functionally characterized the two prognostic groups at the network scale. We overlaid the G1 versus G2 tumors log2 fold-change gene expression on the inferred networks (Data not shown).

We identified that the G1 group expresses genes from two IC41 subnetworks at higher level which are significantly enriched in genes involved in the "innate immune response/interferon 1 response" and "inflammatory response" (Fig 2). Interestingly, IC39, a complex component in terms of function and the most contributive to the G1 group, appears to be related to the epigenetic reprograming of the immune TME (e.g. HDAC11/HDAC6 negatively regulates IL10, KDM6A positively regulates IL6). These observations support conclusions from previous publications about favorable prognostic role of osteosarcoma anti-tumor immunity. In addition, some G1 tumors also express specific cancer testis antigens (CTA; IC26), known as a source of neoantigens. In contrast, the unfavorable prognosis G2 group exhibited connected sub-networks resuming other aspects of the TME previously described as related to poor prognosis and pro-metastatic phenotype such as osteoclast differentiation (IC25), tumor angiogenesis/VEGFR (IC13) and neutrophil degranulation (IC9). Two other isolated subnetworks were strongly upregulated in G2 group (Fig 2): i) The fibroblastic network (IC1) probably reflecting the myofibroblastic reprogramming of osteosarcoma stem cells, crucial for lung metastasis or the cancer-associated fibroblasts (CAF) contributing to mesenchymal-like phenotype and metastasis, and ii) the adipocyte/ PPAR signaling pathway (IC9) involved in inflammatory states and metabolism reprogramming favoring cancer progression. Combined, these G2 characteristics depict an unfavorable osteosarcoma TME prone to induce metastases and imply an early event locking the TME and tumor fate into a vicious cycle.

To complement this functional approach and test the validity of our findings, we returned to the gene expression matrix and performed a differential gene expression analysis between G1/G2 (Fig 2). Consistent with the above results, in G1 tumors we observed the re-expression of specific CTAs, including CTAs from the GAGE family clustered on Xp11.23, and others (including FMR1NB, PASD1, NLRP4, CT45A7, MAGEC3, PAGE2, MAEL, TDRD1, IL13RA2) in addition to genes implicated in spermatogenesis (e.g. RHOXF2B, GTSF1, RHOXF2, DAZ3). This re-expression evokes some major epigenomic changes at methylation level as required in the control of pre-meiosis or meiosis. In accordance, we observed a significant upregulation of some genes (adjusted p-value<$1.3x10^{-4}$) involved in the RNA gene silencing pathway (e.g. MAEL, DDX4, TDRD1), and known to suppress transposable element expression during the meiosis through the piRNA pathway. Similarly, functional characteristics of G2 group tumors were confirmed by the differential gene expression analysis with upregulation of genes implicated in adipocyte differentiation, osteoclastogenesis and angiogenesis, including some link to other G2 functions and poor prognosis in osteosarcoma (e.g. PLA2G2A, PAQR3, HP).

Thus, all three complementary functional analysis methods were consistent, underlying the major contribution of TME to osteosarcoma progression, with innate immune response associated with good prognosis G1 tumors while angiogenesis, osteoclastogenesis and adipogenesis were associated with poor prognosis G2 tumors. This is congruous with the observed clinical efficacy of multityrosine kinase inhibitors with anti-angiogenic activity in relapsed osteosarcoma while the observed inefficacy of zoledronate in front line osteosarcoma treatment is thought to be partially linked to its action on the immune system.

### 1.4. Stratifying ICs are associated with distinct large chromosomic regions

In addition to these distinct TME compositions, we observed altered gene expression involving large chromosomic regions, probably related to CNAs dosage effects (Figure not shown, Table 2) and seemingly associated to each of these G1/G2 stratification groups. The most influential IC contributors to the G1 group (Figure not shown, Table 2) reflected known osteosarcoma cancer cell characteristics such as the cytoband 12q14.1 (CDK4, OS9) either associated with 4qter (IC34) or 6p21.1-22.1 (IC6: RUNX2, CDC5L, UBR2), which contain genes involved in osteosarcoma tumorigenesis and response to chemotherapy. Other G1 group contributive ICs were rather characterized by the dysregulated expression of telomeric regions (IC24: 15qter, 21qter, 12qter; IC34: 4qter; IC35: 13qter). This feature was not detected in the unfavorable G2 group, although the most positively contributive gene of the IC19 was DAXX which, as part of the ATRX/DAXX/HistoneH3.3 complex, regulates telomere maintenance through alternative lengthening of telomeres. For the unfavorable G2 group, three main chromosomic regions were dysregulated on chromosome 6p (IC19), 8q (IC23) and 22q (IC33). The first two are well-known high copy gain or amplification events associated with osteosarcoma oncogenesis more frequent in recurrent/metastatic than in primary osteosarcomas, and previously linked to poor prognosis. Cytoband 6p is a recurrent amplified region in osteosarcoma dysregulating the expression level of the oncogene CDCL5. Cytoband 8q copy number gain is strongly suspected to participate in tumorigenesis through MYC-driven super-enhancer signaling and to be a prognosis factor in osteosarcoma. Surprisingly, chromosome 22q was not previously described as prognostic in osteosarcoma and may require further investigation. These different chromosome imbalances specific to each group might reflect different tumorigenic pathways.

Altogether, those ICs with dysregulated chromosomal regions emphasized a major contribution of CNVs to G1/G2 stratification, even if we cannot exclude that some global modulations might emerge from epigenomic changes. To estimate such potential contribution, we integrated our results with 70 CNV profiles (CGH array) paired with our RNA-sequencing samples. The differential analysis of the aCGH profile using GISTIC2.0 characterized four chromosomes with regions differentially and significantly altered between prognostic groups (adjusted p-value<0.1, Fig 3A). Hence, gains of 2p21-23 and 22q11.22 cytobands and loss of 11p12-p15 cytobands were detected in the G2 group while 13q12 region loss was more pronounced in G1 than G2 group. Among these regions, only the chr22:21859431-22318144 (8.27E-03) region was also identified at the expression level (IC33) as participating to G1/G2 stratification. This region contains PI4KAP2, RIMBP3B, RIMBP3C, UBE2L3, YDJC, CCDC116, SDF2L1, MIR301B, MIR130B, PPIL2, YPEL1, MAPK1, PPM1F and TOP3B genes. The MAPK signaling pathway has been previously proposed as an important driver of the metastatic stage, whereas PI3K-Akt signaling pathway may participate to early and late stages during osteosarcoma evolution. We also noticed that chr8q cytoband, including MYC, was close to significance in aCGH profile analysis, supporting multiple reports about the MYC amplification involvement in osteosarcoma. Considering the number of ICs associated with chromosomal regions and copy number alterations, relatively few of them have been associated with G1/G2 by our analysis, supporting lower contribution of tumor cells to disease progression than TME composition.

### 1.5. Validation of the osteosarcoma RNA-seq prognostic signature

Gene expression in oncology often shows high dispersion between samples and often leads to overfitted models or classifications based on noise rather than signal, questioning result reproducibility in other cohorts as well as the introduction of such models for patient use.

In order to validate the robustness of our stratification in an independent cohort, we identified four gene signatures, based on respectively 37, 15, 8 and 5 genes, predicting G1/G2 tumors by machine learning from initial gene expression count matrix, with logistic regression regularized by elastic-net (Tables 4 to 7), as well as a 20 gene signature predicting G1/G2 tumors by machine learning from initial gene expression count matrix, with logistic regression regularized by LASSO (Table 8).

**Table 4:** 37 genes signature predicting G1/G2 tumors (obtained by machine learning from initial gene expression count matrix, with logistic regression regularized by elastic-net)

| Name | Differential expression |
|---|---|
| Intercept | 0,24599616 |
| AMZ2P1 | -0,0513759 |
| ASXL2 | 0,01629517 |
| BIRC6 | 0,00670994 |
| Cllorf58 | -0,0242489 |
| Clorf53 | -0,0041661 |
| C5orf28 | -0,0537279 |
| CCDC34 | -0,1443061 |
| DCUN1D2 | -0,0056523 |
| DNMT3A | 0,01558531 |
| ECI1 | -0,0175854 |
| EIF3M | -0,0081807 |
| ESCO1 | -0,0479355 |
| FAM35DP | -0,0449686 |
| GAGE12D | -0,0969523 |
| GAGE12G | -0,0299787 |
| HADHA | 0,04567445 |
| HIST2H2AA3 | -0,0327333 |
| HIST2H2AA4 | -0,0327577 |
| MAPK1 | 0,06994424 |
| MEAF6 | -0,1370795 |
| MYO1B | 0,00715753 |
| MYOM3 | 0,03451805 |
| NDP | 0,01211774 |
| PAIP1 | -0,0287883 |
| PCID2 | -0,0039951 |
| PEG10 | -0,0035115 |
| PEX26 | 0,01671823 |
| POLR2C | -0,0163614 |
| PRR14L | 0,001827 |
| RALGAPA2 | 0,03476727 |
| SLC2A6 | 0,06069137 |
| SLC7A4 | 0,04882283 |
| ST7L | -0,0250031 |
| THAP9-AS1 | -0,0575275 |

(continued)

| Name | Differential expression |
|---|---|
| TIMP3 | 0,05513971 |
| TTPAL | 0,03360155 |
| WNT4 | 0,00782038 |

**Table 5:** 15 genes signature predicting G1/G2 tumors (obtained by machine learning from initial gene expression count matrix, with logistic regression regularized by elastic-net)

| Name | Differential expression |
|---|---|
| Intercept | 0,2316098813 |
| AMZ2P1 | -0,0176097649 |
| ASXL2 | 0,0023716918 |
| C11orf58 | -0,0185444892 |
| C5orf28 | -0,0349223925 |
| CCDC34 | -0,0991047579 |
| ESCO1 | -0,0066148013 |
| GAGE12D | -0,0253469115 |
| HADHA | 0,0099403838 |
| MEAF6 | -0,0869212862 |
| PAIP1 | -0,0005712814 |
| RALGAPA2 | 0,0028797811 |
| SLC2A6 | 0,0139299869 |
| SLC7A4 | 0,0106511031 |
| THAP9-AS1 | -0,0168162473 |
| TIMP3 | 0,0076798816 |

**Table 6:** 8 genes signature predicting G1/G2 tumors (obtained by machine learning from initial gene expression count matrix, with logistic regression regularized by elastic-net)

| Name | Differential expression |
|---|---|
| Intercept | 0,2325681 |
| AMZ2P1 | -0,0082083 |
| C5orf28 | -0,0345153 |
| CCDC34 | -0,1512827 |
| GAGE12D | -0,0187314 |
| MEAF6 | -0,130156 |
| SLC2A6 | 0,00826458 |
| SLC7A4 | 0,0048851 |
| THAP9-AS1 | -0,0080192 |

**Table 7:** 2 genes signature predicting G1/G2 tumors (obtained by machine learning from initial gene expression count matrix, with logistic regression regularized by elastic-net)

| Name | Differential expression |
|---|---|
| (Intercept) | 0,22922189 |
| CCDC34 | -0,058127 |
| MEAF6 | -0,0420327 |

**Table 8:** 20 genes signature predicting G1/G2 tumors (obtained by machine learning from initial gene expression count matrix, with logistic regression regularized by LASSO)

| Name | Differential expression |
|---|---|
| (Intercept) | 1,13E+01 |
| AMZ2P1 | -5,51 E-02 |
| C5orf28 | -9,61 E-02 |
| CCDC34 | -5,21 E-01 |
| ESCO1 | -1,73E-01 |
| FAM35DP | -1,43E-01 |
| GAGE12D | -8,48E-02 |
| HADHA | 6,66E-02 |
| HIST2H2AA3 | -7,30E-02 |
| HIST2H2AA4 | -8,69E-16 |
| MAPK1 | 6,26E-01 |
| MEAF6 | -1,28E+00 |
| POLR2C | -1,38E-02 |
| RALGAPA2 | 2,97E-02 |
| SLC2A6 | 1,22E-01 |
| SLC7A4 | 5,16E-02 |
| ST7L | -4,39E-02 |
| THAP9-AS1 | -1,66E-01 |
| TIMP3 | 4,90E-02 |
| TTPAL | 8,42E-02 |

We tested the 15 genes signature shown in Table 5 to predict G1/G2 tumors from an independent cohort of 82 pediatric osteosarcoma tumors whose gene expression count table and paired clinical data are available via open access on the pages regarding the Osteosarcoma project, on the website of the Office of Cancer Genomics (National Cancer Institute). To check prediction validity, we compared OS between predicted G1/G2 and generated related log rank p-values (Fig 3B). Consistent with observations in the OS2006 cohort, results in this independent cohort demonstrated that predicted G2 tumors are significantly associated with worse prognosis than predicted G1 tumors (p-value: 0. 00039), supporting the predictive power of this gene signature, regardless of the first line treatment used. Indeed, in this independent cohort, the first line chemotherapy used, primarily based on the MAP regimen, was different from in the OS2006 cohort. Another valid criticism to RNA-seq based signature, are about reproducibility in laboratory with different technical setup. We therefore designed a custom Nanostring panel based on the RNA-seq signature that we applied to 166 samples from the OS2006 cohort. 70 samples, already analyzed by RNA-seq, were used to retrained our elastic-net model. G1/G2 prediction of the 96 remaining tumors again confirmed that this stratification is associated to significantly different overall and progression free survival (Fig 3C and Fig 6B).

Finally, we used the RNA-seq based signature to estimate the validity of our stratification at relapse to interrogate the reversibility of this prognostic signature throughout disease progression. We observed similar proportions of G1/G2 tumors sampled at diagnosis from the patients in OS2006 RNA-seq cohort who experienced relapse and at relapse from patients in the MAPPYACTS trial (NCT02613962) (Fig 3D). This result supports the persistence of this stratification across disease progression.

### 1.6. Discussion

With unsupervised machine learning strategy, we defined the repertoire of gene components describing osteosarcoma tumor clones and TME. We observed that component interactions through co-expression stratify the cohort into good and poor prognosis tumors. Functional characterization of the components associates good prognosis tumors with specific innate immune expression and poor prognosis tumors with angiogenic, osteoclastic, and adipogenic activities; with distinct CNVs specific to each group.

These distinct functional characteristics enable treatment stratification in osteosarcomas, for instance immune modulation (e.g. mifamurtide) for G1 group tumors, and anti-osteoclastic and anti-angiogenic therapies for G2 group.

We also identified underlying biological pathways of osteosarcoma, involving PPARγ, piRNA or CTAs, which highlight new actionable targets. Our data suggest that early drastic genetic/transcriptomic perturbations in specific clones might influence TME, as well as tumor evolution, response to treatment and metastatic potential.

Finally, we confirmed the predictive power of a minimal prognostic signature of 15 genes within an independent cohort of 82 osteosarcoma primary tumors but also with reproducible Nanostring assay. This work paves the way to the development of prognostic tests, leading to personalized therapy in osteosarcoma, especially by helping clinicians to identify hard-to-treat patients at diagnosis.

Our data highlight that personalized therapies in osteosarcoma should not only be based on genetic abnormalities in the tumor itself (e.g. mutation/CNV), but also on RNA expression profiling to take into account the DNA abnormalities transcribed at RNA level and the TME landscape[22].

## Example 2: Modulation of the PPARγ pathway - a new therapeutic target in osteosarcoma

### Materials and Methods

#### *MTS cell proliferation assay*

HOS, 143B, MG-63, U2OS and IOR/OS18 cell lines were seeded at 5,000 cells per well, Saos-2, Saos-2-B and IOR/OS14 cell lines were seeded at 10,000 cells per well in a 96-well plate containing a final volume of 100 μl/well and left to settle overnight in DMEM with 10% fetal calf serum.

The cells were treated with different drugs (PPARγ agonists troglitazone (TGZ) or rosiglitazone (RGZ), or PPARγ antagonist T0070907, dissolved in DMSO) at concentrations ranging from 0 μmol/l to 100 μmol/L. The control (untreated cells) get the same volume of DMSO when the cells are treated (10μl per 1ml). Cell viability was determined 48 and 72 hours after exposure. Old medium was removed and a solution with MTS/new medium (20 μl of MTS solution - final concentration 0.33 mg/ml) (CellTiter 96 Aqueous One Solution Cell Proliferation Assay; Promega Corporation, Charbonnieres, France) was added. A set of wells were prepared only with MTS/new medium for background subtraction at the same time. An incubation from 1 to 7 hours at 37°C (cell line metabolism dependent) was performed follow by a colorimetric measurement at 490 nm in an automatic plate reader (Elx808; Fisher Bioblock Scientific SAS, Illkirch, France).

#### *Western Blot*

HOS, 143B, MG-63, U2OS, Saos-2, Saos-2-B IOR/OS18 and IOR/OS14 osteosarcoma cell lines were seeded into a 60mm plate dish and collected at approximately 80% confluence. The cells were collected in a lysis buffer (10ml of TNEN 5mM buffer, ½ protease inhibitor pill, 50μl of NaF and 50μl of Orthovanadate (phosphatase inhibitor)) and then obtained by performing an alternation of 5 cycles Frozen in nitrogen and thaw in a water bath at 37°C. Protein supernatants were collected after centrifugation at 4°C for 20min at 13,200rpm.

Protein quantification was performed with the kit BCA of ThermoFisher Scientific (Thermoscientific Pierce TM BCA protein Assay Kit), using a range of bovine serum albumin concentrations (BSA, Euromedex, 04-100-812-E, Souffelweyersheim, France). Absorbance was read at 570 nm using an automatic microplate reader (Elx808; Fisher Bioblock Scientific SAS, Illkirch, France).

Proteins (30μg/well) were separated by 4-20% polyacrylamide gel electrophoresis, tris-glycine extended (Mini-Protean TGX, Bio-Rad, CA, USA), and transferred to polyvinylidene fluoride (PVDF) membranes (Trans-Blot Bio-Rad, CA, USA) using a Trans-Blot Turbo transfer system (Bio-Rad Laboratories, CA, USA).

Membranes were saturated with a 5% BSA for 45 min at room temperature and an antibody against PPARγ (5mg/mL; P5505-05B; US Biological, USA) or against AdipoQ (1:1000; ab75989; Abcam, Cambridge, UK) was added and incubated at 4°C overnight. After, the membranes were washed several times with buffer (TBS 1% and Tween 0.1%) and incubated with a secondary antibody (in goat anti-rabbit IgG, 1:5000; A9169; Sigma-Aldrich, St Louis, MO, USA), at least for 2 hours.

Membranes were revealed using the Clarity Western ECL Substrate kit (Bio-Rad Laboratories, CA, USA), and protein bands detected by chemiluminescence using the Bio-Rad ChemiDoc imaging system (Bio-Rad Laboratories, CA, USA).

A stripping was then performed (stripping buffer 62.5 mL Tris 0.5M, 50 mL SDS 20%, 3.5 mL β-mercaptoethanol, 500 mL H2O) in order to incubate the membranes with the β-actin antibody, directly conjugated to HRP (HRP conjugate; 1:1000; #5125; Cell Signaling, MA, USA) and revealed as described before.

## Results

We studied the therapeutic potential of PPARγ pathway *in vitro* by evaluating cell proliferation under the effect of PPARγ agonists and PPARγ antagonists (MTS test).

The PPARγ antagonist T0070907 decreases the cell viability in 7 human osteosarcoma lines with an IC50 ranging between 9-18μM (median IC50 20μM). With the agonists, an effect is barely observed at the highest concentrations tested, which could be explained by a negative feed-back loop of PPARγ through the expression of a PPARγ negative dominant isoform.

## Conclusion

Modulation of the PPARγ pathway is a new therapeutic target in osteosarcoma.

**Abreviations used in this text:**

| | |
|---|---|
| API-AI | Adriamycin/platinum/ifosfamide |
| AUC | Area under curve |
| BCA | BiCinchoninic acid Assay |
| BSA | Bovine Serum Albumin |
| CAF | Cancer-Associated Fibroblasts |
| CGH | Comparative Genomic Hybridization |
| CNA | Copy Number Aberrations |
| CNV | Copy Number Variation |
| CTA | Cancer Testis Antigens |
| DASL | cDNAmediated Annealing, Selection, and Ligation |
| DMEM | Dulbecco/Vogt modified Eagle's minimal essential medium |
| DMSO | Diméthylsulfoxide |
| DNA | Deoxyribonucleic acid |
| DR | Death Rate |
| dUTP | Deoxyuridine triphosphate |
| FOV | Fields of view |
| HRP | Horse Radish Peroxidase |
| IC | Independent Components |
| ICA | Independent Components Analysis |
| IL | Interleukine |
| MAP | Methotrexate, doxorubicin and cisplatinum |
| MCR | Minimum common region |
| M-EI | MTX-etoposide/ifosfamide |
| OLA | Oligonucleotide Ligation Assay |
| OS | Overall survival |
| OTS | Osteosarcoma |
| PCA | Principal Component Analysis |
| PFER | Per-family error rate |
| PFS | Progression-free survival |

| | |
|---|---|
| PPARγ | Peroxisome proliferator-activated receptor |
| PVDF | Polyvinylidene fluoride |
| qRT-PCR | Quantitative reverse-transcription polymerase chain reaction |
| RGZ | Rosiglitazone |
| RNA | Ribonucleic acid |
| RNA-seq | RNA-sequencing |
| TGZ | Troglitazone |
| TME | Tumor microenvironment |
| WES | Whole Exome Sequencing |
| WGS | Whole Genome Sequencing |

**REFERENCES**

1. Perry, J. A. et al. Complementary genomic approaches highlight the PI3K/mTOR pathway as a common vulnerability in osteosarcoma. Proc. Natl. Acad. Sci. 111, E5564-E5573 (2014).

2. Lorenz, S. et al. Unscrambling the genomic chaos of osteosarcoma reveals extensive transcript fusion, recurrent rearrangements and frequent novel TP53 aberrations. Oncotarget 7, (2016).

3. Trama, A. et al. Survival of European adolescents and young adults diagnosed with cancer in 2000-07: population-based data from EUROCARE-5. Lancet Oncol. (2016) doi:10.1016/S1470-2045(16)00162-5.

4. Bielack, S. S. et al. Methotrexate, Doxorubicin, and Cisplatin (MAP) Plus Maintenance Pegylated Interferon Alfa-2b Versus MAP Alone in Patients With Resectable High-Grade Osteosarcoma and Good Histologic Response to Preoperative MAP: First Results of the EURAMOS-1 Good Response Randomized Controlled Trial. J. Clin. Oncol. 33, 2279-2287 (2015).

5. Neyssa M Marina et al. Comparison of MAPIE versus MAP in patients with a poor response to preoperative chemotherapy for newly diagnosed high-grade osteosarcoma (EURAMOS-1): an open-label, international, randomised controlled trial. Lancet Oncol 1396-1408 (2016).

6. Piperno-Neumann, S. et al. Zoledronate in combination with chemotherapy and surgery to treat osteosarcoma (OS2006): a randomised, multicentre, open-label, phase 3 trial. Lancet Oncol. 17, 1070-1080 (2016).

7. Meyers, P. A. et al. Osteosarcoma: The Addition of Muramyl Tripeptide to Chemotherapy Improves Overall Survival--A Report From the Children's Oncology Group. J. Clin. Oncol. 26, 633-638 (2008).

8. Ozaki, T. et al. Genetic imbalances revealed by comparative genomic hybridization in osteosarcomas. Int. J. Cancer 102, 355-365 (2002).

9. Man, T.-K. et al. Genome-wide array comparative genomic hybridization analysis reveals distinct amplifications in osteosarcoma. BMC Cancer 4, 1 (2004).

10. Atiye, J. et al. Gene amplifications in osteosarcoma-CGH microarray analysis: CGH Microarray Analysis of Osteosarcoma. Genes. Chromosomes Cancer 42, 158-163 (2005).

11. Selvarajah, S. et al. Genomic signatures of chromosomal instability and osteosarcoma progression detected by high resolution array CGH and interphase FISH. Cytogenet. Genome Res. 122, 5-15 (2008).

12. Fletcher. Identification of chromosomal aberrations associated with disease progression and a novel 3q13.31 deletion involving LSAMP gene in osteosarcoma. Int. J. Oncol. 35, (2009).

13. Kovac, M. et al. Exome sequencing of osteosarcoma reveals mutation signatures reminiscent of BRCA deficiency. Nat. Commun. 6, 8940 (2015).

14. Bousquet, M. et al. Whole-exome sequencing in osteosarcoma reveals important heterogeneity of genetic alterations. Ann. Oncol. 27, 738-744 (2016).

15. Chen, K. S. et al. A novel TP53-KPNA3 translocation defines a de novo treatment-resistant clone in osteosarcoma. Mol. Case Stud. 2, a000992 (2016).

16. Chiappetta, C. et al. Whole-exome analysis in osteosarcoma to identify a personalized therapy. Oncotarget 8, 80416 (2017).

17. Chen, X. et al. Recurrent Somatic Structural Variations Contribute to Tumorigenesis in Pediatric Osteosarcoma. Cell Rep. 7, 104-112 (2014).

18. Ribi, S., Baumhoer, D. & Lee, K. TP53 intron 1 hotspot rearrangements are specific to sporadic osteosarcoma and can cause Li-Fraumeni syndrome. Oncotarget 6, 7727 (2015).

19. Behjati, S. et al. Recurrent mutation of IGF signalling genes and distinct patterns of genomic rearrangement in osteosarcoma. Nat. Commun. 8, 15936 (2017).

20. Gambera, S. et al. Clonal dynamics in osteosarcoma defined by RGB marking. Nat. Commun. 9, (2018).

21. Petitprez, F. et al. Quantitative Analyses of the Tumor Microenvironment Composition and Orientation in the Era of Precision Medicine. Front. Oncol. 8, 390 (2018).

22. Quail, D. F. & Joyce, J. A. Microenvironmental regulation of tumor progression and metastasis. Nat. Med. 19, 1423-1437 (2013).

23. Kairov, U. et al. Determining the optimal number of independent components for reproducible transcriptomic data analysis. BMC Genomics 18, 712 (2017).

24. Liberzon, A. et al. Molecular signatures database (MSigDB) 3.0. Bioinformatics 27, 1739-1740 (2011).

25. Raudvere, U. et al. g:Profiler: a web server for functional enrichment analysis and conversions of gene lists (2019 update). Nucleic Acids Res. 47, W191-W198 (2019).

26. Cheng, F. et al. Divergent roles of histone deacetylase 6 (HDAC6) and histone deacetylase 11 (HDAC11) on the transcriptional regulation of IL10 in antigen presenting cells. Mol. Immunol. 60, 44-53 (2014).

27. de Groot, A. E. & Pienta, K. J. Epigenetic control of macrophage polarization: implications for targeting tumor-associated macrophages. Oncotarget 9, (2018).

28. Buddingh, E. P. et al. Tumor-Infiltrating Macrophages Are Associated with Metastasis Suppression in High-Grade Osteosarcoma: A Rationale for Treatment with Macrophage Activating Agents. Clin. Cancer Res. 17, 2110-2119 (2011).

29. Gomez-Brouchet A et al. CD163-positive tumor-associated macrophages and CD8-positive cytotoxic lymphocytes are powerful diagnostic markers for the therapeutic stratification of osteosarcoma patients: An immunohistochemical analysis of the biopsies from the French OS2006 phase 3 trial. Oncoimmunology. Aug 24, doi: 10.1080/2162402X.2017.1331193. (2017).

30. Salmaninejad, A. et al. Cancer/Testis Antigens: Expression, Regulation, Tumor Invasion, and Use in Immunotherapy of Cancers. Immunol. Invest. 45, 619-640 (2016).

31. Kelleher, F. C. & O'Sullivan, H. Monocytes, Macrophages, and Osteoclasts in Osteosarcoma. J. Adolesc. Young Adult Oncol. (2017) doi:10.1089/jayao.2016.0078.

32. Yang, J. et al. Genetic amplification of the vascular endothelial growth factor (VEGF) pathway genes, including VEGFA , in human osteosarcoma: VEGFA Amplification in Osteosarcoma. Cancer 117, 4925-4938 (2011).

33. Mollinedo, F. Neutrophil Degranulation, Plasticity, and Cancer Metastasis. Trends Immunol. 40, 228-242 (2019).

34. Zhang, W. et al. Adaptive Fibrogenic Reprogramming of Osteosarcoma Stem Cells Promotes Metastatic Growth. Cell Rep. 24, 1266-1277.e5 (2018).

35. Liu, Y. et al. The Role of PPAR-δ in Metabolism, Inflammation, and Cancer: Many Characters of a Critical Transcription Factor. Int. J. Mol. Sci. 19, 3339 (2018).

36. Fratta, E. et al. The biology of cancer testis antigens: Putative function, regulation and therapeutic potential. Mol. Oncol. 5, 164-182 (2011).

37. McFarlane, R. J. & Wakeman, J. A. Meiosis-like Functions in Oncogenesis: A New View of Cancer. Cancer Res. 77, 5712-5716 (2017).

38. Ozata, D. M., Gainetdinov, I., Zoch, A., O'Carroll, D. & Zamore, P. D. PIWI-interacting RNAs: small RNAs with big functions. Nat. Rev. Genet. 20, 89-108 (2019).

39. Mintz, M. B. et al. An Expression Signature Classifies Chemotherapy-Resistant Pediatric Osteosarcoma. Cancer Res 8 (2005).

40. Ma, Z. et al. The tumor suppressor role of PAQR3 in osteosarcoma. Tumor Biol. 36, 3319-3324 (2015).

41. Arredouani, M. et al. Haptoglobin directly affects T cells and suppresses T helper cell type 2 cytokine release. Immunology 108, 144-151 (2003).

42. Kwon, J.-O. et al. Haptoglobin Acts as a TLR4 Ligand to Suppress Osteoclastogenesis via the TLR4-IFN-β Axis. J. Immunol. ji1800661 (2019) doi:10.4049/jimmunol.1800661.

43. Duffaud, F. et al. Efficacy and safety of regorafenib in adult patients with metastatic osteosarcoma: a non-comparative, randomised, double-blind, placebo-controlled, phase 2 study. Lancet Oncol. 20, 120-133 (2019).

44. Mannerström, B. et al. Epigenetic alterations in mesenchymal stem cells by osteosarcoma-derived extracellular vesicles. Epigenetics 14, 352-364 (2019).

45. Lu, X.-Y. et al. Cell cycle regulator gene CDC5L, a potential target for 6p12-p21 amplicon in osteosarcoma. Mol. Cancer Res. 6, 937-946 (2008).

46. Martin, J. W. et al. Digital Expression Profiling Identifies RUNX2, CDC5L, MDM2, RECQL4, and CDK4 as Potential Predictive Biomarkers for Neo-Adjuvant Chemotherapy Response in Paediatric Osteosarcoma. PLoS ONE 9, e95843 (2014).

47. Hoelper, D., Huang, H., Jain, A. Y., Patel, D. J. & Lewis, P. W. Structural and mechanistic insights into ATRX-dependent and -independent functions of the histone chaperone DAXX. Nat. Commun. 8, 1193 (2017).

48. Lau, C. C. et al. Frequent amplification and rearrangement of chromosomal bands 6p12-p21 and 17p11.2 in osteosarcoma. Genes. Chromosomes Cancer 39, 11-21 (2004).

49. Chen, D. et al. Super enhancer inhibitors suppress MYC driven transcriptional amplification and tumor progression in osteosarcoma. Bone Res. 6, (2018).

50. Tarkkanen, M. et al. DNA sequence copy number increase at 8q: A potential new prognostic marker in high-grade osteosarcoma. Int. J. Cancer 84, 114-121 (1999).

51. Wang, D. et al. Multiregion Sequencing Reveals the Genetic Heterogeneity and Evolutionary History of Osteosarcoma and Matched Pulmonary Metastases. Cancer Res. 79, 7-20 (2019).

52. Gaspar, N. et al. Results of methotrexate-etoposide-ifosfamide based regimen (M-EI) in osteosarcoma patients included in the French OS2006/sarcome-09 study. Eur. J. Cancer 88, 57-66 (2018).

53. Piperno-Neumann, S. et al. Results of API-AI based regimen in osteosarcoma adult patients included in the French OS2006/Sarcome-09 study: Results of API-AI based regimen in osteosarcoma adult patients included in the French OS2006/Sarcome-09 study. Int. J. Cancer (2019) doi:10.1002/ijc.32526.

54. Shannon, P. Cytoscape: A Software Environment for Integrated Models of Biomolecular Interaction Networks. Genome Res. 13, 2498-2504 (2003).

55. Haw, R., Loney, F., Ong, E., He, Y. & Wu, G. Perform Pathway Enrichment Analysis Using ReactomeFIViz. in vol. 2074: 165-179. doi: 10.1007/978-1-4939-9873-9_13. PubMed PMID: 31583638 (2020).

56. Le Cao, K.-A., Rohart, F., Gonzalez, I. & Dejean, S. mixOmics: Omics Data Integration Project. R package version 6.1.1. (2016).

57. Love, M. I., Huber, W. & Anders, S. Moderated estimation of fold change and dispersion for RNA-seq data with DESeq2. Genome Biol. 15, (2014).

58. Love, M. I., Huber, W. & Anders, S. Moderated estimation of fold change and dispersion for RNA-seq data with DESeq2. Genome Biol. 15, 550 (2014).

59. Friedman, J., Hastie, T. & Tibshirani, R. Regularization Paths for Generalized Linear Models via Coordinate Descent. J. Stat. Softw. 33, (2010).

60. Picard, F. et al. Joint segmentation, calling, and normalization of multiple CGH profiles. Biostatistics 12, 413-428 (2011).

61. Nilsen, G. et al. Copynumber: Efficient algorithms for single- and multi-track copy number segmentation. BMC Genomics 13, 591 (2012).

62. Karolchik, D. The UCSC Genome Browser Database. Nucleic Acids Res. 31, 51-54 (2003).

63. Gaujoux, R. & Seoighe, C. A flexible R package for nonnegative matrix factorization. BMC Bioinformatics 11, 367 (2010).

64. Hornik, K., Feinerer, I., Kober, M. & Buchta, C. Spherical k-Means Clustering. J. Stat. Softw. 22.

65. Mermel, C. H. et al. GISTIC2.0 facilitates sensitive and confident localization of the targets of focal somatic copy-number alteration in human cancers. Genome Biol. 12, R41 (2011).

66. Jiang P. et al. Genome-Scale Signatures of Gene Interaction from Compound Screens Predict Clinical Efficacy of Targeted Cancer Therapies. Cell.Syst. Mar 28;6(3):343-354.e5.(2018).

67. Liu X. et al. A prognostic gene expression signature for oropharyngeal squamous cell carcinoma. EBioMedicine. Nov;61:102805 (2020).

68. Vey, J. et al. A Toolbox for Functional Analysis and the Systematic Identification of Diagnostic and Prognostic Gene Expression Signatures Combining Meta-Analysis and Machine Learning. Cancer. Oct21;11(10):1606 (2019).

69. Xia Y. et al. Genetic determinants of the molecular portraits of epithelial cancers. Nat.Commun. Dec 11;10(1):5666 (2019).

## Claims

1. An *in vitro* method for determining prognosis of osteosarcoma in an subject, comprising the steps of:

   (a) measuring expression levels of a collection of signature genes from a biological sample taken from said subject, wherein said collection of signature genes comprises at least two genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, GAGE12G, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4; and
   (b) applying the expression levels measured in step (a) to a predictive model relating expression levels of said collection of signature genes with osteosarcoma outcome; and
   (c) evaluating the output of said predictive model to determine prognosis of osteosarcoma in said subject.

2. The *in vitro* method of claim 1, wherein said collection of signature genes comprises CCDC34 and MEAF6.

3. The *in vitro* method of any of claims 1 or claim 2, wherein said collection of signature genes comprises AMZ2P1, C5orf28, CCDC34, GAGE12D, MEAF6, SLC2A6, SLC7A4 and THAP9-AS1.

4. The *in vitro* method of any of claims 1 to 3, wherein said collection of signature genes comprises AMZ2P1, C5orf28, CCDC34, ESCO1, GAGE12D, HADHA, MEAF6, RALGAPA2, SLC2A6, SLC7A4, THAP9-AS1 and TIMP3.

5. The *in vitro* method of any of claims 1 to 4, wherein said collection of signature genes comprises AMZ2P1, ASXL2, C11orf58, C5orf28, CCDC34, ESCO1, GAGE12D, HADHA, MEAF6, PAIP1, RALGAPA2, SLC2A6, SLC7A4, THAP9-AS1 and TIMP3.

6. The *in vitro* method of any of claims 1 to 5, wherein said collection of signature genes comprises AMZ2P1, C5orf28, CCDC34, ESCO1, FAM35DP, GAGE12D, HADHA, HIST2H2AA3, HIST2H2AA4, MAPK1, MEAF6, POLR2C, RALGAPA2, SLC2A6, SLC7A4, ST7L, THAP9-AS1, TIMP3 and TTPAL.

7. The method of any of the preceding claims, wherein the expression levels of said collection of signature genes are measured at diagnosis.

8. The method of any of the preceding claims, wherein the expression levels of said collection of signature genes are measured at relapse.

9. The method of any of the preceding claims, further comprising combining the gene expression levels of said signature genes with one or more other parameters to predict progression of osteosarcoma in said subject.

10. The method of any of the preceding claims, wherein the biological sample is an osteosarcoma biopsy sample from the subject.

11. A diagnostic kit for predicting progression of osteosarcoma in a subject, wherein said kit comprises at least one nucleic acid probe or oligonucleotide, which can be used in a method as defined in any one of claims 1 to 10 for measuring the level of expression of a collection of signature genes from a biological sample taken from said subject, wherein said collection of signature genes comprises at least two genes selected from the group consisting of: AMZ2P1, ASXL2, BIRC6, C11orf58, C1orf53, C5orf28, CCDC34, DCUN1D2, DNMT3A, ECI1, EIF3M, ESCO1, FAM35DP, GAGE12D, HADHA, HIST2H2AA3, HIST2H2AA4, MAPK1, MEAF6, MYO1B, MYOM3, NDP, PAIP1, PCID2, PEG10, PEX26, POLR2C, PRR14L, RALGAPA2, SLC2A6, SLC7A4, ST7L, THAP9-AS1, TIMP3, TTPAL, WNT4.

12. A PPARγ inhibitor for use as an antineoplastic treatment in a subject with osteosarcoma.

13. The PPARγ inhibitor of claim 12, for use according to claim 12, wherein said PPARγ inhibitor is T0070907 (CAS N°313516-66-4).

14. The PPARγ inhibitor of claim 12, for use according to claim 12 or 13, wherein said subject is an adolescent or a young adult.

15. The PPARγ inhibitor of claim 12, for use according to any of claims 12-14 wherein said subject has been identified as a poor responder to chemotherapy by the method of any of claims 1 to 10.

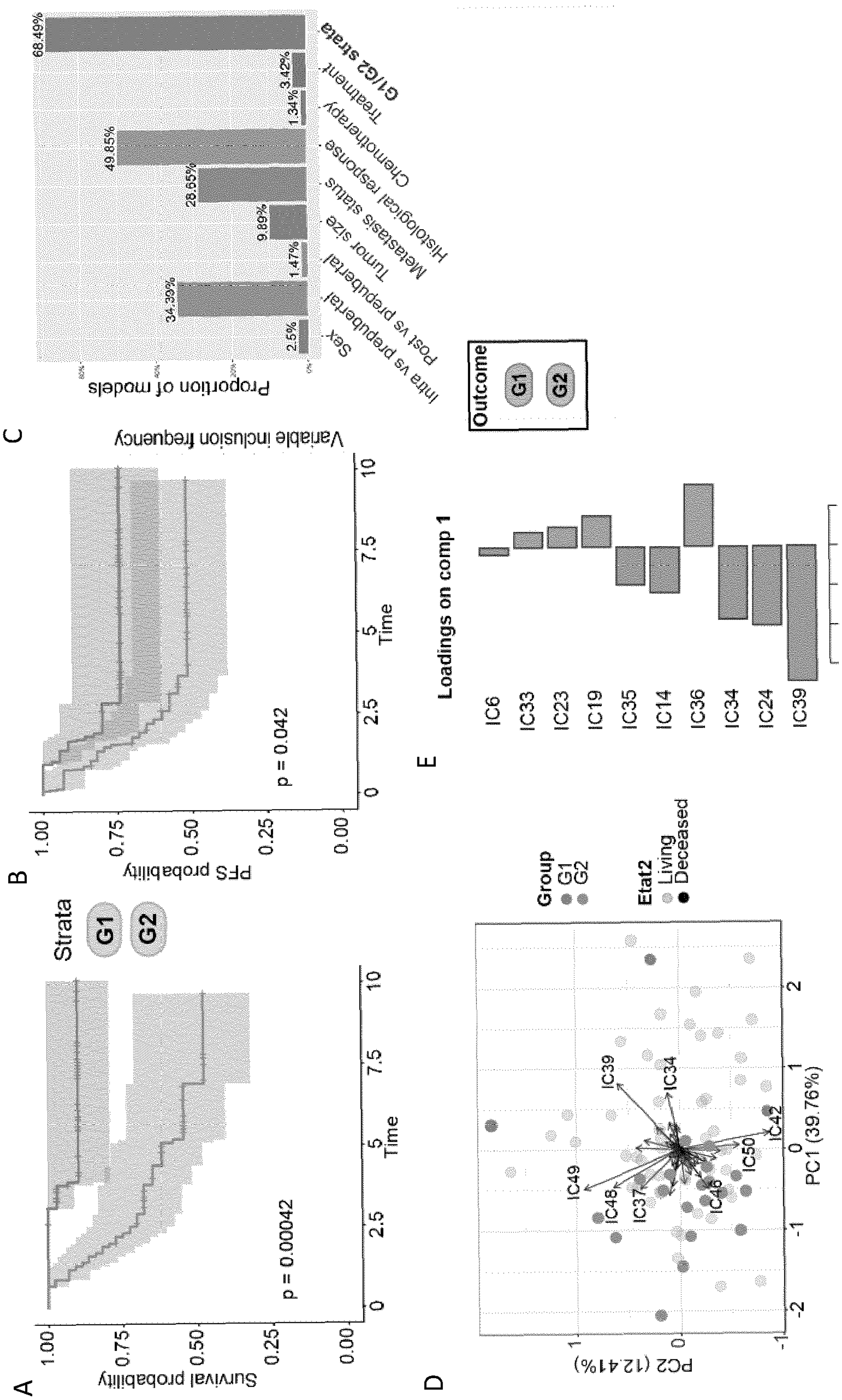
A
Survival probability
p = 0.00042
Time
Strata
G1
G2
B
PFS probability
p = 0.042
Time
C
Variable inclusion frequency
Proportion of models
Sex
Intra vs prepubertal
Post vs prepubertal
Tumor size
Metastasis status
Histological response
Chemotherapy
Treatment
G1/G2 strata
2.5%
34.39%
1.47%
9.89%
28.55%
49.85%
1.34%
3.42%
68.49%
Outcome
G1
G2
D
PC1 (39.76%)
PC2 (12.41%)
Group
G1
G2
Etat2
Living
Deceased
E
Loadings on comp 1
IC6
IC33
IC23
IC19
IC35
IC14
IC36
IC34
IC24
IC39

Figure 1

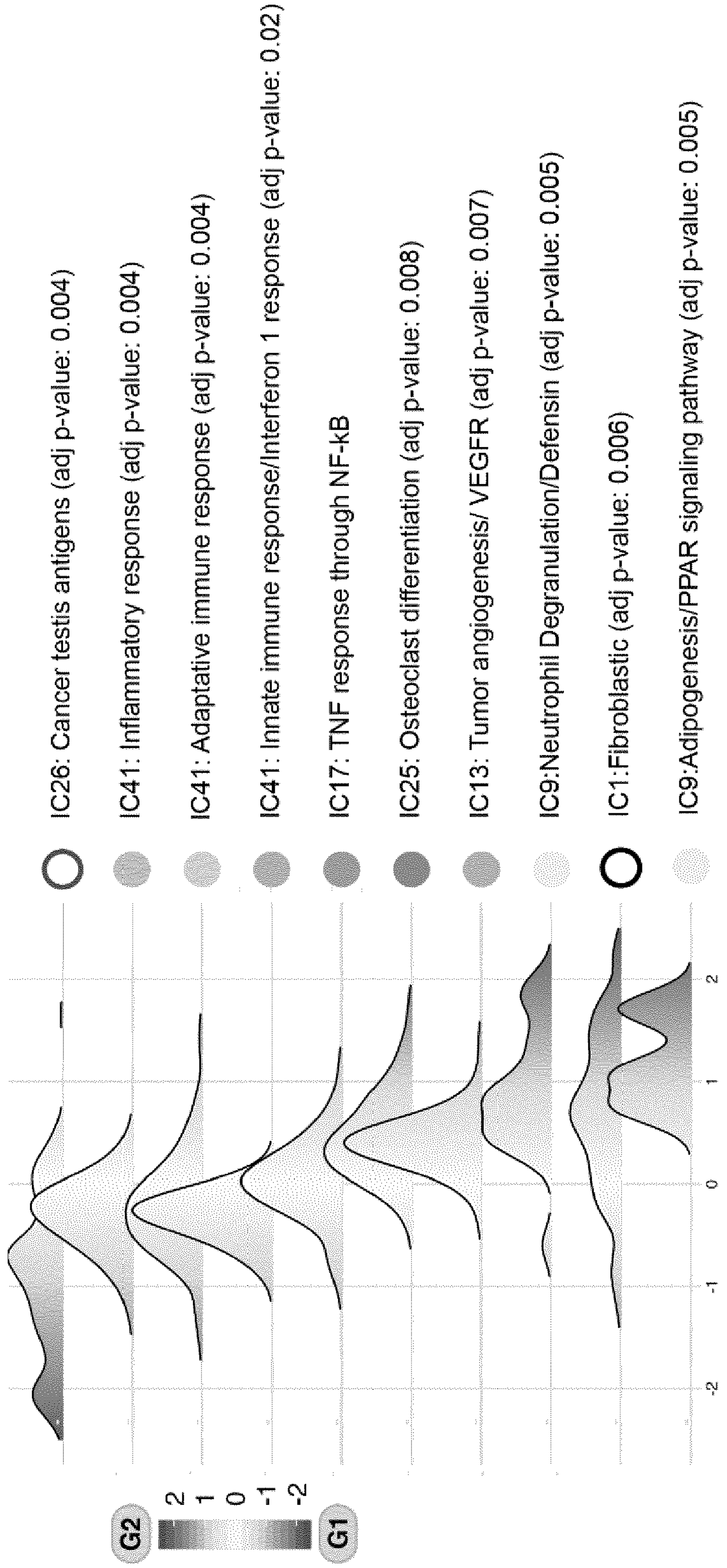
G2
2
1
0
-1
-2
G1
-2
-1
0
1
2
log2FoldChange
IC26: Cancer testis antigens (adj p-value: 0.004)
IC41: Inflammatory response (adj p-value: 0.004)
IC41: Adaptative immune response (adj p-value: 0.004)
IC41: Innate immune response/Interferon 1 response (adj p-value: 0.02)
IC17: TNF response through NF-kB
IC25: Osteoclast differentiation (adj p-value: 0.008)
IC13: Tumor angiogenesis/ VEGFR (adj p-value: 0.007)
IC9:Neutrophil Degranulation/Defensin (adj p-value: 0.005)
IC1:Fibroblastic (adj p-value: 0.006)
IC9:Adipogenesis/PPAR signaling pathway (adj p-value: 0.005)

**Figure 2**

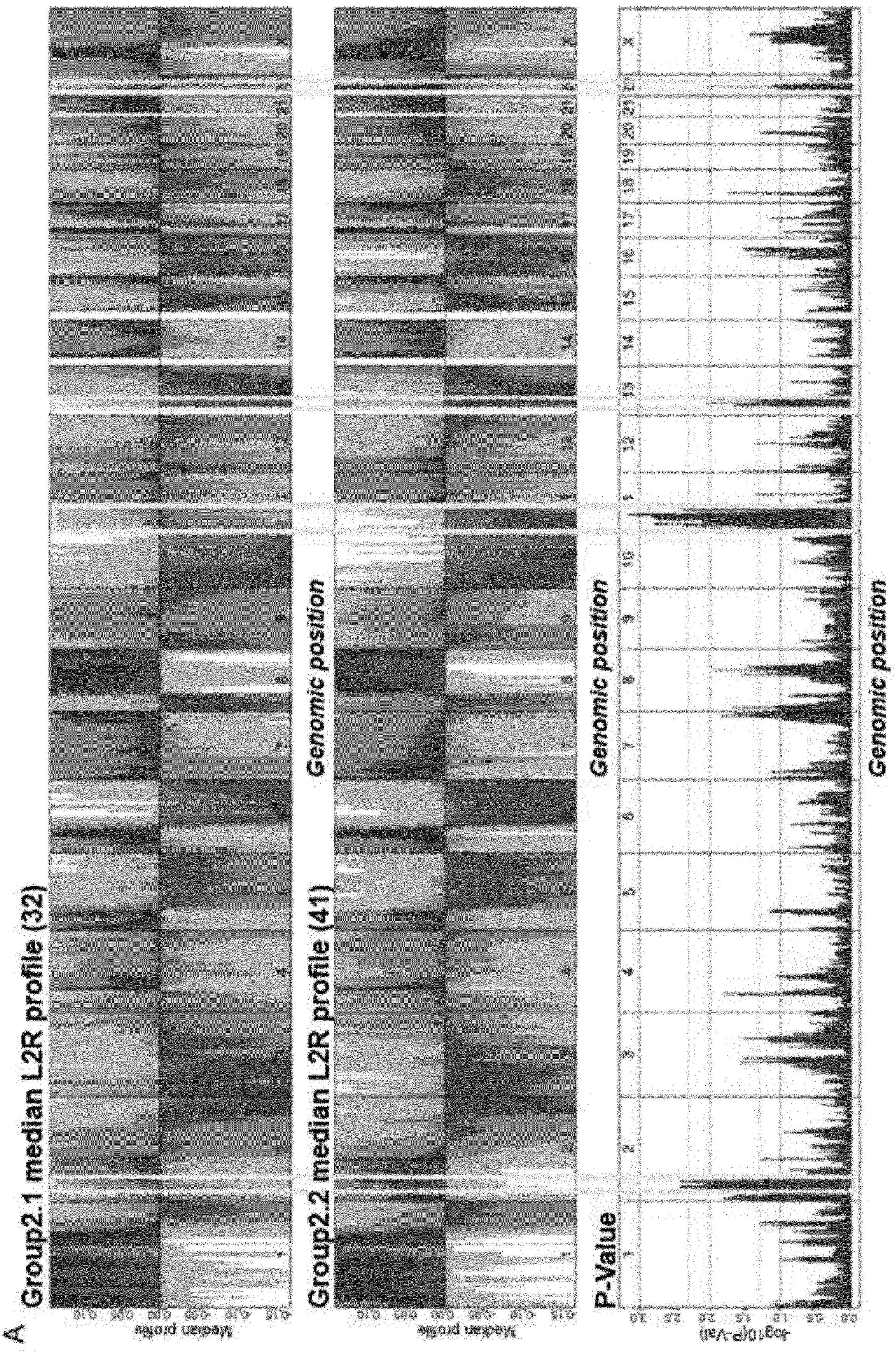
A
Group2.1 median L2R profile (32)
Median profile
Genomic position
Group2.2 median L2R profile (41)
Median profile
Genomic position
P-Value
-log10(P-Val)
Genomic position

Figure 3A

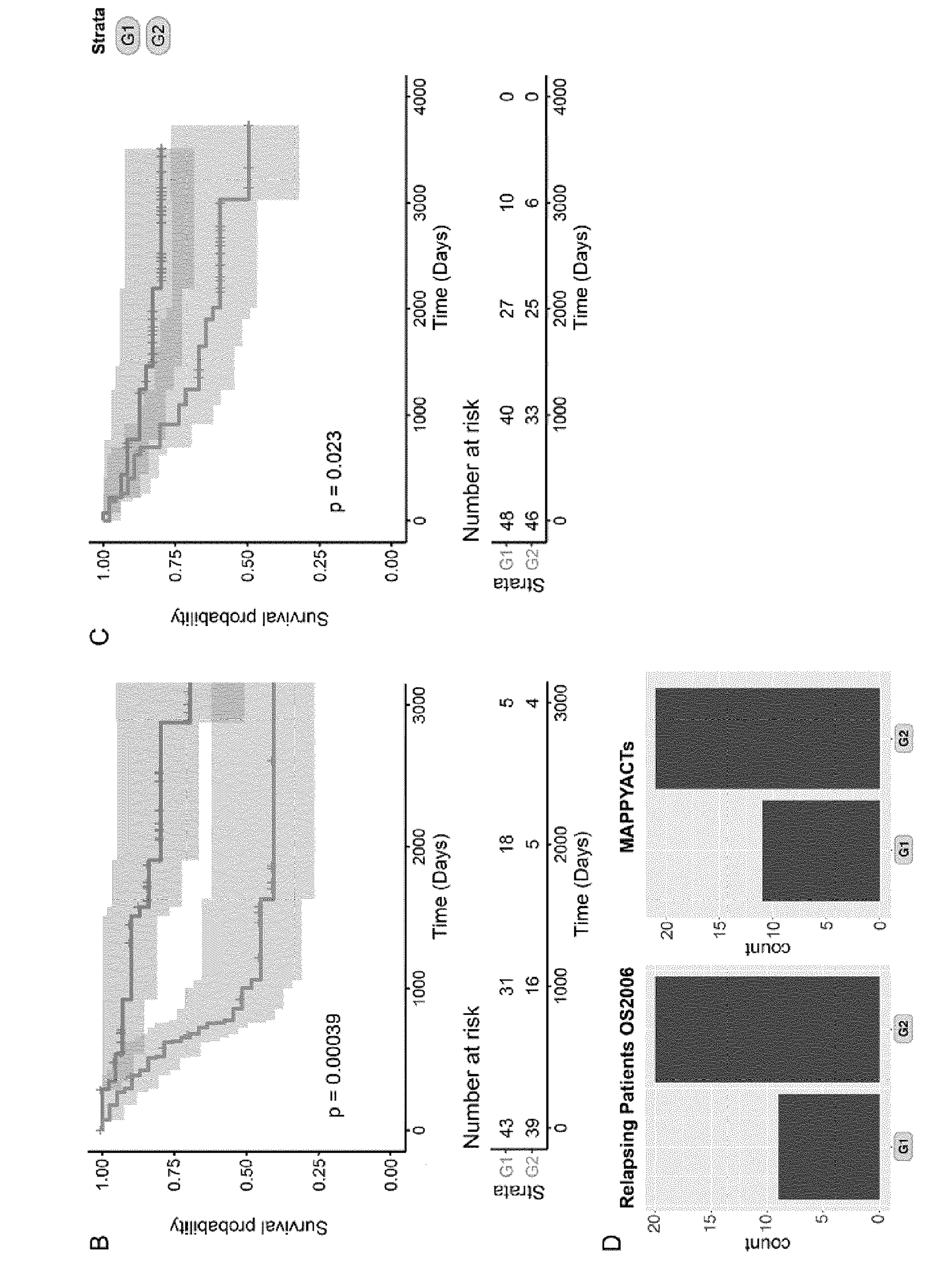
B
Survival probability
1.00
0.75
0.50
0.25
0.00
p = 0.00039
0
1000
2000
3000
Time (Days)
Number at risk
Strata
G1
G2
43
31
18
5
39
16
5
4
C
p = 0.023
4000
48
40
27
10
0
46
33
25
6
0
Strata
G1
G2
D
Relapsing Patients OS2006
MAPPYACTs
count
20
15
10
5
0

Figure 3B-C-D

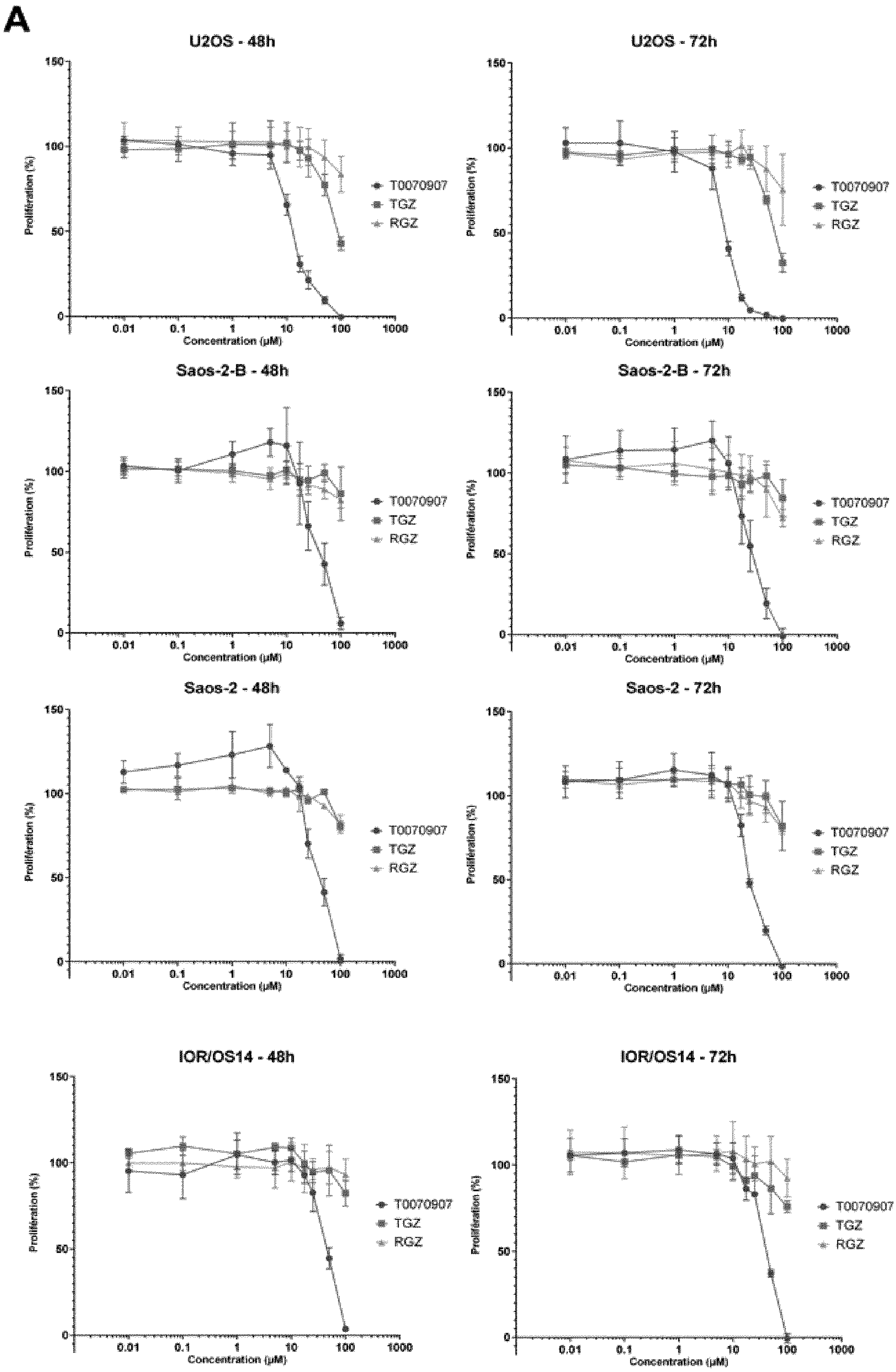


Figure 4A (beginning)

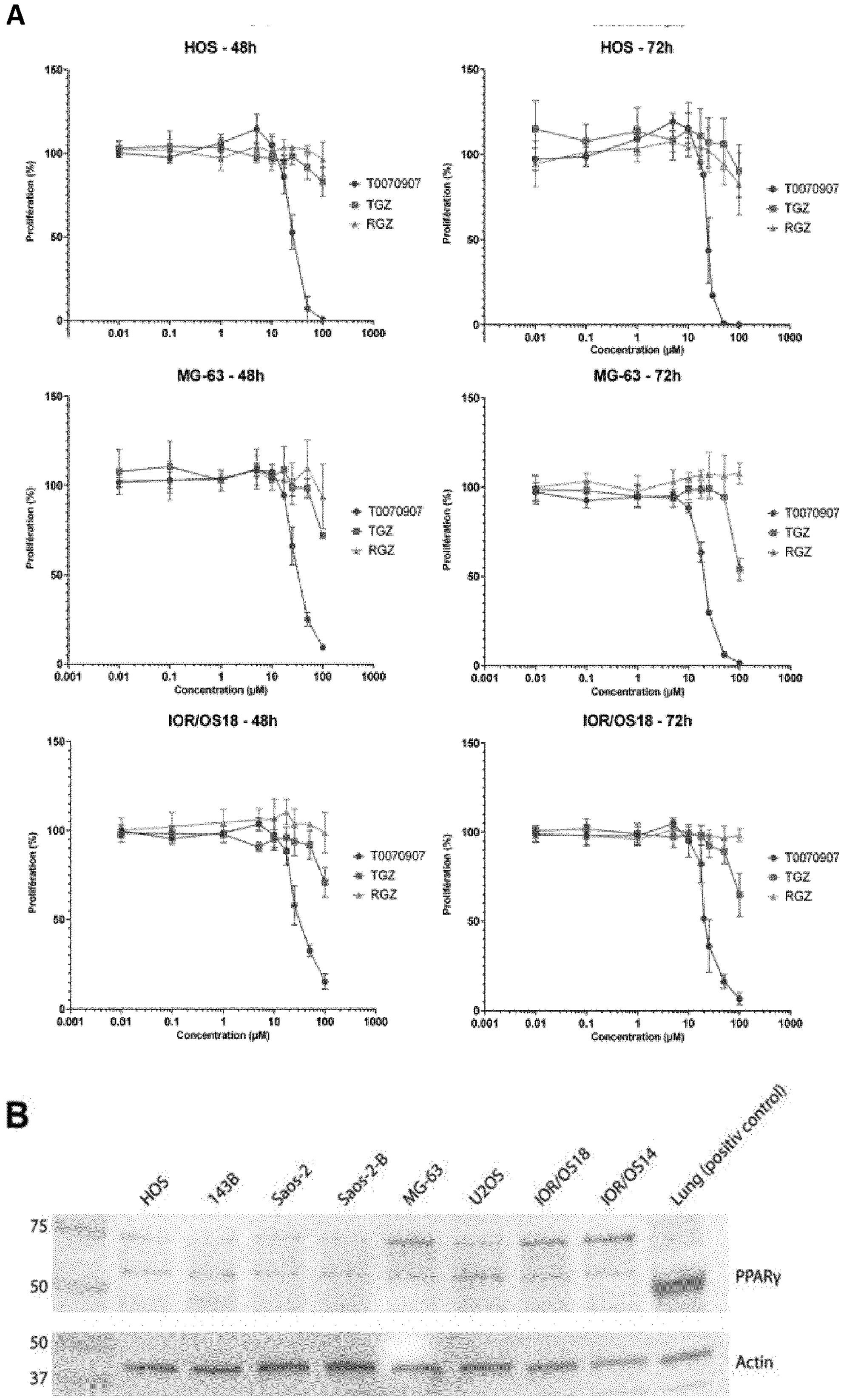


**Figure 4A (follow-up), 4B**

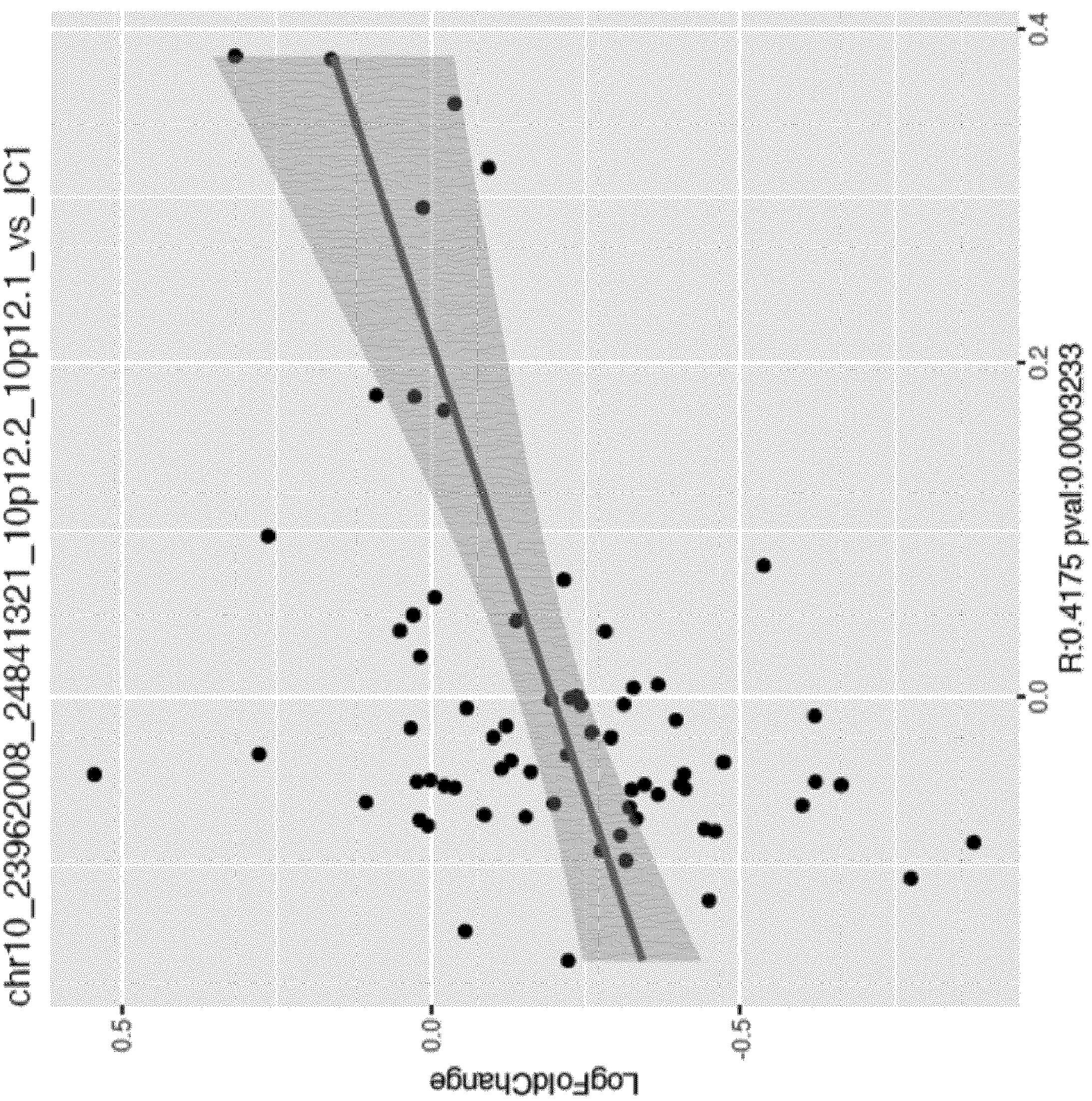
chr10_239620008_24841321_10p12.2_10p12.1_vs_IC1
LogFoldChange
0.5
0.0
-0.5
0.0
0.2
0.4
R:0.4175 pval:0.0003233

**Figure 5**

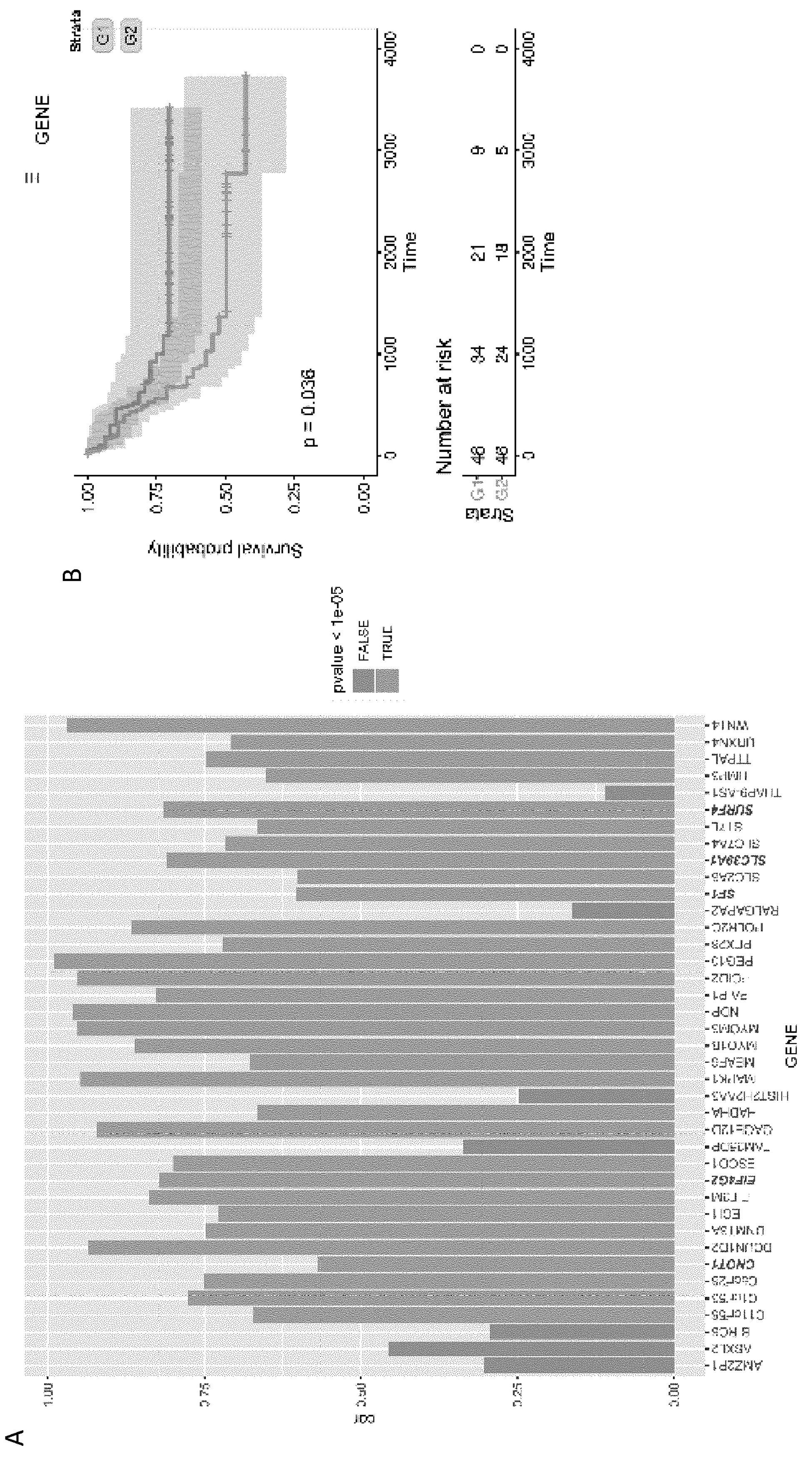
A
B
pvalue < 1e-05
FALSE
TRUE
GENE
Survival probability
p = 0.036
Time
Number at risk
Strata
G1
G2
48
34
21
9
0
24
18
5


Figure 6

Europäisches Patentamt
European Patent Office
Office européen des brevets

# EUROPEAN SEARCH REPORT

Application Number
EP 21 30 5080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FAN HONGWU ET AL: "Identification of critical genes associated with human osteosarcoma metastasis based on integrated gene expression profiling", MOLECULAR MEDICINE REPORTS, 3 June 2019 (2019-06-03), XP055815742, GR ISSN: 1791-2997, DOI: 10.3892/mmr.2019.10323 * the whole document * ----- | 1-11 | INV. C12Q1/6886 |
| X | OUYANG ZHANBO ET AL: "Construction of a Five-Super-Enhancer-Associated-Genes Prognostic Model for Osteosarcoma Patients", FRONTIERS IN CELL AND DEVELOPMENTAL BIOLOGY, vol. 8, 1 January 2020 (2020-01-01), page 598660, XP55816194, DOI: 10.3389/fcell.2020.598660 Retrieved from the Internet: URL:https://www.ncbi.nlm.nih.gov/pmc/articles/PMC7661850/pdf/fcell-08-598660.pdf> * the whole document * ----- | 11 | TECHNICAL FIELDS SEARCHED (IPC) C12Q |
| X | CN 112 063 720 A (SHANGHAI GENERAL HOSPITAL) 11 December 2020 (2020-12-11) * the whole document * ----- | 1,7-11 | |
| X | WO 2018/132899 A1 (UTI LP [CA]) 26 July 2018 (2018-07-26) * the whole document * ----- -/-- | 12-15 | |

The present search report has been drawn up for all claims

1

| Place of search | Date of completion of the search | Examiner |
|---|---|---|
| The Hague | 22 June 2021 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 2

Europäisches Patentamt
European Patent Office
Office européen des brevets

# EUROPEAN SEARCH REPORT

Application Number
EP 21 30 5080

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GU WEI ET AL: "The analgesic effects of pioglitazone in the bone cancer pain rats via regulating the PPAR[gamma]/PTEN/mTOR signaling pathway in the spinal dorsal horn", BIOMEDICINE AND PHARMACOTHERAPY, ELSEVIER, FR, vol. 131, 14 September 2020 (2020-09-14), XP086322454, ISSN: 0753-3322, DOI: 10.1016/J.BIOPHA.2020.110692 [retrieved on 2020-09-14] * the whole document * ----- | 12-15 | |
| | | | TECHNICAL FIELDS SEARCHED (IPC) |

1

The present search report has been drawn up for all claims

| Place of search | Date of completion of the search | Examiner |
|---|---|---|
| The Hague | 22 June 2021 | Cornelis, Karen |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 21 30 5080

This annex lists the patent family members relating to the patent documents cited in the above-mentioned European search report.
The members are as contained in the European Patent Office EDP file on
The European Patent Office is in no way liable for these particulars which are merely given for the purpose of information.

22-06-2021

| Patent document cited in search report | Publication date | Patent family member(s) | Publication date |
|---|---|---|---|
| CN 112063720 A | 11-12-2020 | NONE | |
| WO 2018132899 A1 | 26-07-2018 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VEY et al.** *Cancers,* 2019
- **XIA et al.** *Nature Communications,* 2019
- **JIANG et al.** *Cell Systems,* 2018
- **LIU et al.** *EBioMedicine,* 2020
- **HOFNER et al.** Controlling false discoveries in high-dimensional situations: boosting with stability selection. *BMC Bioinformatics,* 2015, vol. 16 (144
- Stability selection. **MEINSHAUSEN ; BÜHLMANN.** J. R. Statist. Soc. B. Royal Statistical Society 1369-7412/10/72417, 2010, vol. 72, 417-473
- **PERRY, J. A. et al.** Complementary genomic approaches highlight the PI3K/mTOR pathway as a common vulnerability in osteosarcoma. *Proc. Natl. Acad. Sci.,* 2014, vol. 111, E5564-E5573
- **LORENZ, S. et al.** Unscrambling the genomic chaos of osteosarcoma reveals extensive transcript fusion, recurrent rearrangements and frequent novel TP53 aberrations. *Oncotarget,* 2016, vol. 7
- **TRAMA, A. et al.** Survival of European adolescents and young adults diagnosed with cancer in 2000-07: population-based data from EUROCARE-5. *Lancet Oncol,* 2016
- **BIELACK, S. S. et al.** Methotrexate, Doxorubicin, and Cisplatin (MAP) Plus Maintenance Pegylated Interferon Alfa-2b Versus MAP Alone in Patients With Resectable High-Grade Osteosarcoma and Good Histologic Response to Preoperative MAP: First Results of the EURAMOS-1 Good Response Randomized Controlled Trial. *J. Clin. Oncol.,* 2015, vol. 33, 2279-2287
- **NEYSSA M MARINA et al.** Comparison of MAPIE versus MAP in patients with a poor response to preoperative chemotherapy for newly diagnosed high-grade osteosarcoma (EURAMOS-1): an open-label, international, randomised controlled trial. *Lancet Oncol,* 2016, 1396-1408
- **PIPERNO-NEUMANN, S. et al.** Zoledronate in combination with chemotherapy and surgery to treat osteosarcoma (OS2006): a randomised, multicentre, open-label, phase 3 trial. *Lancet Oncol.,* 2016, vol. 17, 1070-1080
- **MEYERS, P. A. et al.** Osteosarcoma: The Addition of Muramyl Tripeptide to Chemotherapy Improves Overall Survival--A Report From the Children's Oncology Group. *J. Clin. Oncol.,* 2008, vol. 26, 633-638
- **OZAKI, T. et al.** Genetic imbalances revealed by comparative genomic hybridization in osteosarcomas. *Int. J. Cancer,* 2002, vol. 102, 355-365
- **MAN, T.-K. et al.** Genome-wide array comparative genomic hybridization analysis reveals distinct amplifications in osteosarcoma. *BMC Cancer,* 2004, vol. 4 (1
- **ATIYE, J. et al.** Gene amplifications in osteosarcoma-CGH microarray analysis: CGH Microarray Analysis of Osteosarcoma. *Genes. Chromosomes Cancer,* 2005, vol. 42, 158-163
- **SELVARAJAH, S. et al.** Genomic signatures of chromosomal instability and osteosarcoma progression detected by high resolution array CGH and interphase FISH. Cytogenet. *Genome Res.,* 2008, vol. 122, 5-15
- **FLETCHER.** Identification of chromosomal aberrations associated with disease progression and a novel 3q13.31 deletion involving LSAMP gene in osteosarcoma. *Int. J. Oncol.,* 2009, vol. 35
- **KOVAC, M. et al.** Exome sequencing of osteosarcoma reveals mutation signatures reminiscent of BRCA deficiency. *Nat. Commun.,* 2015, vol. 6, 8940
- **BOUSQUET, M. et al.** Whole-exome sequencing in osteosarcoma reveals important heterogeneity of genetic alterations. *Ann. Oncol.,* 2016, vol. 27, 738-744
- **CHEN, K. S. et al.** A novel TP53-KPNA3 translocation defines a de novo treatment-resistant clone in osteosarcoma. *Mol. Case Stud.,* 2016, vol. 2, a000992
- **CHIAPPETTA, C. et al.** Whole-exome analysis in osteosarcoma to identify a personalized therapy. *Oncotarget,* 2017, vol. 8, 80416
- **CHEN, X. et al.** Recurrent Somatic Structural Variations Contribute to Tumorigenesis in Pediatric Osteosarcoma. *Cell Rep.,* 2014, vol. 7, 104-112
- **RIBI, S. ; BAUMHOER, D. ; LEE, K.** TP53 intron 1 hotspot rearrangements are specific to sporadic osteosarcoma and can cause Li-Fraumeni syndrome. *Oncotarget,* 2015, vol. 6, 7727
- **BEHJATI, S. et al.** Recurrent mutation of IGF signalling genes and distinct patterns of genomic rearrangement in osteosarcoma. *Nat. Commun.,* 2017, vol. 8, 15936
- **GAMBERA, S. et al.** Clonal dynamics in osteosarcoma defined by RGB marking. *Nat. Commun,* 2018, vol. 9

- **PETITPREZ, F. et al.** Quantitative Analyses of the Tumor Microenvironment Composition and Orientation in the Era of Precision Medicine. *Front. Oncol.,* 2018, vol. 8, 390
- **QUAIL, D. F. ; JOYCE, J. A.** Microenvironmental regulation of tumor progression and metastasis. *Nat. Med.,* 2013, vol. 19, 1423-1437
- **KAIROV, U. et al.** Determining the optimal number of independent components for reproducible transcriptomic data analysis. *BMC Genomics,* 2017, vol. 8, 712
- **LIBERZON, A. et al.** Molecular signatures database (MSigDB) 3.0. *Bioinformatics,* 2011, vol. 27, 1739-1740
- **RAUDVERE, U. et al.** g:Profiler: a web server for functional enrichment analysis and conversions of gene lists (2019 update). *Nucleic Acids Res.,* 2019, vol. 47, W191-W198
- **CHENG, F. et al.** Divergent roles of histone deacetylase 6 (HDAC6) and histone deacetylase 11 (HDAC11) on the transcriptional regulation of IL10 in antigen presenting cells. *Mol. Immunol.,* 2014, vol. 60, 44-53
- **DE GROOT, A. E. ; PIENTA, K. J.** Epigenetic control of macrophage polarization: implications for targeting tumor-associated macrophages. *Oncotarget,* 2018, vol. 9
- **BUDDINGH, E. P. et al.** Tumor-Infiltrating Macrophages Are Associated with Metastasis Suppression in High-Grade Osteosarcoma: A Rationale for Treatment with Macrophage Activating Agents. *Clin. Cancer Res.,* 2011, vol. 17, 2110-2119
- **GOMEZ-BROUCHET A et al.** CD163-positive tumor-associated macrophages and CD8-positive cytotoxic lymphocytes are powerful diagnostic markers for the therapeutic stratification of osteosarcoma patients: An immunohistochemical analysis of the biopsies from the French OS2006 phase 3 trial. *Oncoimmunology,* 24 August 2017
- **SALMANINEJAD, A. et al.** Cancer/Testis Antigens: Expression, Regulation, Tumor Invasion, and Use in Immunotherapy of Cancers. *Immunol. Invest.,* 2016, vol. 45, 619-640
- **KELLEHER, F. C. ; O'SULLIVAN ; H. MONOCYTES, MACROPHAGES ; OSTEOCLASTS.** *Osteosarcoma. J. Adolesc. Young Adult Oncol.,* 2017
- **YANG, J. et al.** Genetic amplification of the vascular endothelial growth factor (VEGF) pathway genes, including VEGFA , in human osteosarcoma: VEGFA Amplification in Osteosarcoma. *Cancer,* 2011, vol. 117, 4925-4938
- **MOLLINEDO, F.** Neutrophil Degranulation, Plasticity, and Cancer Metastasis. *Trends Immunol.,* 2019, vol. 40, 228-242
- **ZHANG, W. et al.** Adaptive Fibrogenic Reprogramming of Osteosarcoma Stem Cells Promotes Metastatic Growth. *Cell Rep.,* 2018, vol. 24, 1266-1277.e5
- **LIU, Y. et al.** The Role of PPAR-δ in Metabolism, Inflammation, and Cancer: Many Characters of a Critical Transcription Factor. *Int. J. Mol. Sci.,* 2018, vol. 19, 3339
- **FRATTA, E. et al.** The biology of cancer testis antigens: Putative function, regulation and therapeutic potential. *Mol. Oncol.,* 2011, vol. 5, 164-182
- **MCFARLANE, R. J. ; WAKEMAN, J. A.** Meiosis-like Functions in Oncogenesis: A New View of Cancer. *Cancer Res.,* 2017, vol. 77, 5712-5716
- **OZATA, D. M. ; GAINETDINOV, I. ; ZOCH, A. ; O'CARROLL, D. ; ZAMORE, P. D.** PIWI-interacting RNAs: small RNAs with big functions. *Nat. Rev. Genet.,* 2019, vol. 20, 89-108
- **MINTZ, M. B. et al.** An Expression Signature Classifies Chemotherapy-Resistant Pediatric Osteosarcoma. *Cancer Res,* 2005, vol. 8
- **MA, Z. et al.** The tumor suppressor role of PAQR3 in osteosarcoma. *Tumor Biol,* 2015, vol. 36, 3319-3324
- **ARREDOUANI, M. et al.** Haptoglobin directly affects T cells and suppresses T helper cell type 2 cytokine release. *Immunology,* 2003, vol. 108, 144-151
- **KWON, J.-O et al.** Haptoglobin Acts as a TLR4 Ligand to Suppress Osteoclastogenesis via the TLR4-IFN-β Axis. *J. Immunol.,* 2019
- **DUFFAUD, F. et al.** Efficacy and safety of regorafenib in adult patients with metastatic osteosarcoma: a non-comparative, randomised, double-blind, placebo-controlled, phase 2 study. *Lancet Oncol.,* 2019, vol. 20, 120-133
- **MANNERSTRÖM, B. et al.** Epigenetic alterations in mesenchymal stem cells by osteosarcoma-derived extracellular vesicles. *Epigenetics,* 2019, vol. 14, 352-364
- **LU, X.-Y. et al.** Cell cycle regulator gene CDC5L, a potential target for 6p12-p21 amplicon in osteosarcoma. *Mol. Cancer Res.,* 2008, vol. 6, 937-946
- **MARTIN, J. W. et al.** Digital Expression Profiling Identifies RUNX2, CDC5L, MDM2, RECQL4, and CDK4 as Potential Predictive Biomarkers for Neo-Adjuvant Chemotherapy Response in Paediatric Osteosarcoma. *PLoS ONE,* 2014, vol. 9, e95843
- **HOELPER, D. ; HUANG, H. ; JAIN, A. Y. ; PATEL, D. J. ; LEWIS, P. W.** Structural and mechanistic insights into ATRX-dependent and -independent functions of the histone chaperone DAXX. *Nat. Commun,* 2017, vol. 8, 1193
- **LAU, C. C. et al.** Frequent amplification and rearrangement of chromosomal bands 6p12-p21 and 17p11.2 in osteosarcoma. *Genes. Chromosomes Cancer,* 2004, vol. 39, 11-21

- **CHEN, D. et al.** Super enhancer inhibitors suppress MYC driven transcriptional amplification and tumor progression in osteosarcoma. *Bone Res.,* 2018, vol. 6
- **TARKKANEN, M. et al.** DNA sequence copy number increase at 8q: A potential new prognostic marker in high-grade osteosarcoma. *Int. J. Cancer,* 1999, vol. 84, 114-121
- **WANG, D. et al.** Multiregion Sequencing Reveals the Genetic Heterogeneity and Evolutionary History of Osteosarcoma and Matched Pulmonary Metastases. *Cancer Res.,* 2019, vol. 79, 7-20
- **GASPAR, N. et al.** Results of methotrexate-etoposide-ifosfamide based regimen (M-EI) in osteosarcoma patients included in the French OS2006/sarcome-09 study. *Eur. J. Cancer,* 2018, vol. 88, 57-66
- **PIPERNO-NEUMANN, S. et al.** Results of API-AI based regimen in osteosarcoma adult patients included in the French OS2006/Sarcome-09 study: Results of API-AI based regimen in osteosarcoma adult patients included in the French OS2006/Sarcome-09 study. *Int. J. Cancer,* 2019
- **SHANNON, P. CYTOSCAPE.** A Software Environment for Integrated Models of Biomolecular Interaction Networks. *Genome Res.,* 2003, vol. 13, 2498-2504
- **HAW, R. ; LONEY, F. ; ONG, E. ; HE, Y. ; WU, G.** *Perform Pathway Enrichment Analysis Using ReactomeFIViz,* vol. 2074, 165-179
- **LE CAO, K.-A. ; ROHART, F. ; GONZALEZ, I. ; DEJEAN, S.** *mixOmics: Omics Data Integration Project. R package version 6.1.1.,* 2016
- **LOVE, M. I. ; HUBER, W. ; ANDERS, S.** Moderated estimation of fold change and dispersion for RNA-seq data with DESeq2. *Genome Biol.,* 2014, vol. 15
- **LOVE, M. I. ; HUBER, W. ; ANDERS, S.** Moderated estimation of fold change and dispersion for RNA-seq data with DESeq2. *Genome Biol.,* 2014, vol. 15, 550
- **FRIEDMAN, J. ; HASTIE, T. ; TIBSHIRANI, R.** Regularization Paths for Generalized Linear Models via Coordinate Descent. *J. Stat. Softw.,* 2010, vol. 33
- **PICARD, F. et al.** Joint segmentation, calling, and normalization of multiple CGH profiles. *Biostatistics,* 2011, vol. 12, 413-428
- **NILSEN, G. et al.** Copynumber: Efficient algorithms for single- and multi-track copy number segmentation. *BMC Genomics,* 2012, vol. 13, 591
- **KAROLCHIK, D.** The UCSC Genome Browser Database. *Nucleic Acids Res.,* 2003, vol. 31, 51-54
- **GAUJOUX, R. ; SEOIGHE, C.** A flexible R package for nonnegative matrix factorization. *BMC Bioinformatics,* 2010, vol. 11, 367
- **HORNIK, K. ; FEINERER, I. ; KOBER, M. ; BUCHTA, C.** Spherical k-Means Clustering. *J. Stat. Softw.,* vol. 22
- **MERMEL, C. H. et al.** GISTIC2.0 facilitates sensitive and confident localization of the targets of focal somatic copy-number alteration in human cancers. *Genome Biol.,* 2011, vol. 12, R41
- **JIANG P. et al.** Genome-Scale Signatures of Gene Interaction from Compound Screens Predict Clinical Efficacy of Targeted Cancer Therapies. *Cell.Syst.,* 28 March 2018, vol. 6 (3), 343-354.e5
- **LIU X. et al.** A prognostic gene expression signature for oropharyngeal squamous cell carcinoma. *EBioMedicine,* November 2020, vol. 61, 102805
- **VEY, J. et al.** A Toolbox for Functional Analysis and the Systematic Identification of Diagnostic and Prognostic Gene Expression Signatures Combining Meta-Analysis and Machine Learning. *Cancer,* 21 October 2019, vol. 11 (10), 1606
- **XIA Y. et al.** Genetic determinants of the molecular portraits of epithelial cancers. *Nat.Commun,* 11 December 2019, vol. ;10 (1), 5666

SEQUENCE LISTING

<110> INSTITUT GUSTAVE ROUSSY

<120> METHOD FOR IDENTIFYING HARD-TO-TREAT OSTEOSARCOMA PATIENTS AT DIAGNOSIS AND IMPROVING THEIR OUTCOME BY PROVIDING NEW THERAPY

<130> B200148EPA

<160> 41

<170> PatentIn version 3.5

<210> 1
<211> 100
<212> DNA
<213> Homo sapiens

<400> 1
aactcttttg aactaagatt tgcctaggtg aataaattgg ggatgacggt tgtctggaga 60

agatataagg tccatttgtg aatactctgc actatgagac 100

<210> 2
<211> 100
<212> DNA
<213> Homo sapiens

<400> 2
ccagcattga cacacaccaa taccacgaag gactaagtaa agcaacccaa gatcagatcc 60

ttcagactct cattcagagg gttcggaggc agaatcttct 100

<210> 3
<211> 100
<212> DNA
<213> Homo sapiens

<400> 3
gtgagcacac acagaatgtg ccattgtcag tcactcttgc aacaagtcct gcacagtttc 60

cttgtacgga tggaactgac agaatatctt gctttgggtc 100

<210> 4
<211> 100
<212> DNA
<213> Homo sapiens

<400> 4
agtccagatg attctgaaag cgattcagag tcagagaaag aagaatctgc tgaagaactc 60

caagctgctg agcaccctga tgaagtggag gatcccaaaa 100

<210> 5
<211> 100
<212> DNA
<213> Homo sapiens

<400> 5

taaactatgt ggatccagct actggctatg tggtgctcac acagattgcc cacttgcaaa 60

gaggtgaatg ttgtggctct gcgtgcagac attgtccata 100

<210> 6
<211> 100
<212> DNA
<213> Homo sapiens

<400> 6
aatgcgaaac ataaacctcg tccagctgca aagagctatg gttatgccaa tggaaaactt 60

acaggttttt acagtggaaa ttcctatcca gaaccagcct 100

<210> 7
<211> 100
<212> DNA
<213> Homo sapiens

<400> 7
gcaggcagtc accaagtctg tatttctagg cctgaccttt tggatcctta tggcaagtgc 60

ctccaagtct gcaaatctaa aacaacccat tttgcccatt 100

<210> 8
<211> 100
<212> DNA
<213> Homo sapiens

<400> 8
ggcacggtcc tgaggcagac aggcatttat tacgctgcct attttcgcat gtggatttca 60

gtggcgatgg taaaagcagt ggcaaagatt tccatcagac 100

<210> 9
<211> 100
<212> DNA
<213> Homo sapiens

<400> 9
gcaacacggc atgtgtcctc tgcagggcgc tgcgttttta tacacgtcaa agctgttaag 60

aatgtgccct aagggagagg atcttgtcgt agagtctaat 100

<210> 10
<211> 100
<212> DNA
<213> Homo sapiens

<400> 10
ggcgagagga ctggccctcc cggctccaga tgttcttcgc taataaccac gaccaggaat 60

ttgaccctcc aaaggtttac ccacctgtcc cagctgagaa 100

<210> 11
<211> 100
<212> DNA

<213> Homo sapiens

<400> 11
acgcagcgcg atgcggacgt gcagaacttc gtcagcttca tctccaaaga ctccatccag 60

aagtccctgc agatgtactt agagaggctc aaagaagaaa 100

<210> 12
<211> 100
<212> DNA
<213> Homo sapiens

<400> 12
gaagagtgat gctgcttcaa aagtcatggt ggaattgctc ggaagttaca cagaggacaa 60

tgcttcccag gctcgagttg atgcccacag gtgtattgta 100

<210> 13
<211> 100
<212> DNA
<213> Homo sapiens

<400> 13
tatgtttgga caaatgccag gtagcggaat tggtactggt ccaggagtta tccaggatag 60

attttcaccc accatgggac gtcatcgttc aaatcaactc 100

<210> 14
<211> 100
<212> DNA
<213> Homo sapiens

<400> 14
ttctcccaaa gggaaaaaac gcaaggtaga acatcagaca gcttgtgctt gtagttctca 60

atgcacgcaa ggatctgaaa agtgtcctca gaagactact 100

<210> 15
<211> 100
<212> DNA
<213> Homo sapiens

<400> 15
cctattattg gcctagacca aggcgctatg tacagcctcc tgaaatgatt gggcctatgc 60

ggcccgagca gttcagtgat gaagtggaac cagcaacacc 100

<210> 16
<211> 100
<212> DNA
<213> Homo sapiens

<400> 16
gggcgagtgc atcgcttgct gcgcaaaggc aactacgcgg agcgagtggg ggccggcgcg 60

cccgtctaca tggctgcggt cctcgagtat ctgaccgccg 100

<210> 17
<211> 100
<212> DNA
<213> Homo sapiens

<400> 17
ccatcttcag caacttgact gggcagcttg attaccaagg ttttgaaaag gccgacatgg 60

tgattgaagc tgtgtttgag gaccttagtc ttaagcacag 100

<210> 18
<211> 100
<212> DNA
<213> Homo sapiens

<400> 18
actgccagag aaccctgagg gagataaaaa tcttactgcg cttcagacat gagaacatca 60

ttggaatcaa tgacattatt cgagcaccaa ccatcgagca 100

<210> 19
<211> 100
<212> DNA
<213> Homo sapiens

<400> 19
atttggagcg acagatctat gcttttgagg gaagctacct ggaagacact cagatgtatg 60

gcaatattat tcgtggctgg gatcggtatc tgaccaacca 100

<210> 20
<211> 100
<212> DNA
<213> Homo sapiens

<400> 20
tatgcgcaac aaaagaggta ccagcagaca aagagttccg ccttagtaat tcagtcttat 60

atccggggtt ggaaggctcg aaaaattctg cgggaactga 100

<210> 21
<211> 100
<212> DNA
<213> Homo sapiens

<400> 21
aagttatctc cagccgctga gctacatcta atcttcaaca acaaggagat cttcagctcg 60

ccgaaccgca aaatcaattt tgaccgagag aagggcctgg 100

<210> 22
<211> 100
<212> DNA
<213> Homo sapiens

<400> 22
agaaaacatg tactagctgc atccttttct atgctctccc tgctggtgat aatgggagat 60

acagacagta aaacggacag ctcattcata atggactcgg 100

<210> 23
<211> 100
<212> DNA
<213> Homo sapiens

<400> 23
ttgaacagtt tgcagagacc ctgaatggtt gtgttacaac agatgatgct ttgcaagaac 60

ttgtggaact catctatcaa caggccacat ctatcccaaa 100

<210> 24
<211> 100
<212> DNA
<213> Homo sapiens

<400> 24
tactgaaaac acaccagctg tctctggatg cttttctggt tgccttgaag ttcatgcagg 60

tggaggacgt ggacattgac gaagttcagt gtattctggc 100

<210> 25
<211> 100
<212> DNA
<213> Homo sapiens

<400> 25
tttgccacca ctgcaagcaa aagtctggag aagttcacca acgacaagaa cgattaggga 60

aaatatgctg ctgtgggtta acaactcaga aagtccctga 100

<210> 26
<211> 100
<212> DNA
<213> Homo sapiens

<400> 26
gtttggggac agggactgtg tccatgaaac attccatctt cttggtgaag gcaaggggtt 60

ggttcttcag gtcaggatgt taatggagct ggaagttcag 100

<210> 27
<211> 100
<212> DNA
<213> Homo sapiens

<400> 27
gacatcagta ccaactagaa gtgggtcata gatagattac cagggatgca gtggtgttta 60

ggcaggatag gtctttactg gccctgactg ctgttaataa 100

<210> 28
<211> 100
<212> DNA
<213> Homo sapiens

<400> 28
cgctgtgctc agattcttct cagggtcaaa aggcagaaaa cacacagaat tcgagttctt 60

cagagcctca gcctattcaa gagaataaag gacatgtgaa 100

<210> 29
<211> 100
<212> DNA
<213> Homo sapiens

<400> 29
caggagaaga tgagccactt catgccctgg ttactgccaa tacaatggag aacgtcaaaa 60

aggcagtgga acagataaga aacatcctga agcagggtat 100

<210> 30
<211> 100
<212> DNA
<213> Homo sapiens

<400> 30
agggtgaaca gtatagactt tgtagaattg gagcaccttc aacctgatgt gttagtccac 60

gcagtactaa gagttgttga tttcctatac tgacagaggc 100

<210> 31
<211> 100
<212> DNA
<213> Homo sapiens

<400> 31
gggcgcgggt cgggaccctg cagaacaaaa gggtgttcct ggccaccttc gccgcagtgc 60

tcggcaattt cagctttggg tatgccctgg tctacacatc 100

<210> 32
<211> 100
<212> DNA
<213> Homo sapiens

<400> 32
agagacatat caggggacat taggattggg gaagacactt gactgctaga atcagaggtt 60

ggacactata cataaggaca ggctcacatg ggaggctgga 100

<210> 33
<211> 100
<212> DNA
<213> Homo sapiens

<400> 33
gcctcatgct gaaacttagc tatctgacct gggtgcgctt ctccatctgg ctgctgatgg 60

gacttgcagt gtatttcggc tatggcatcc ggcatagcaa 100

<210> 34
<211> 100

<212> DNA
<213> Homo sapiens

<400> 34
aacctgtcag ctcaggacca tcagaccttt ttcacctgtg acacagattt tttacgtcct 60

tcagacacag ttatgcagaa ggcttggagg gaaagaaatc 100

<210> 35
<211> 100
<212> DNA
<213> Homo sapiens

<400> 35
gaatattggt aagatcgagc aatggcttca ggacatgggt tctcttctcc tgtgatcatt 60

caagtgctca ctgcatgaag actggcttgt ctcagtgttt 100

<210> 36
<211> 100
<212> DNA
<213> Homo sapiens

<400> 36
gctttagagg cgcgggactt ccacacttcc tctccgaagg ccctgcgccg gtgccgtagc 60

caatgacagg ccacgttgtg tcccagcagc caatcggaag 100

<210> 37
<211> 100
<212> DNA
<213> Homo sapiens

<400> 37
acctgccttg ctttgtgact tccaagaacg agtgtctctg gaccgacatg ctctccaatt 60

tcggttaccc tggctaccag tccaaacact acgcctgcat 100

<210> 38
<211> 100
<212> DNA
<213> Homo sapiens

<400> 38
gcatccgaga ctgaaaagac ccatgcttta ctgcagactt gtagcactga atctcttatt 60

tccagccttg gtctgggggc attttgcctc gtagctgaca 100

<210> 39
<211> 100
<212> DNA
<213> Homo sapiens

<400> 39
actgggagtt tgtgtttttc acaggaaccc taagtataga cctctgctgc tcatcaggaa 60

acttactgga gatgaaggcc ccagctgttg tcaccgggtt 100

```
<210>  40
<211>  100
<212>  DNA
<213>  Homo sapiens

<400>  40
catcgcgacg gccaaaagga gcggcgcggt cttcgtggtg ttcgtggcag gtgatgatga       60

acagtctaca cagatggctg caagttggga agatgataaa                           100


<210>  41
<211>  100
<212>  DNA
<213>  Homo sapiens

<400>  41
gggtcagccc acagggcttc cagtggtcag gatgctctga caacatcgcc tacggtgtgg       60

ccttctcaca gtcgtttgtg gatgtgcggg agagaagcaa                           100
```